(19) Europäisches Patentamt
European Patent Office
Office européen des brevets

(11) EP 0 550 450 B1

(12) EUROPEAN PATENT SPECIFICATION

(45) Date of publication and mention of the grant of the patent:
**14.05.2003 Bulletin 2003/20**

(21) Application number: **91914256.2**

(22) Date of filing: **30.07.1991**

(51) Int Cl.7: **C12N 11/00**, C12P 21/02

(86) International application number:
**PCT/US91/05415**

(87) International publication number:
**WO 92/002617 (20.02.1992 Gazette 1992/05)**

# (54) USE OF CROSSLINKED CRYSTALS AS A NOVEL FORM OF ENZYME IMMOBILIZATION

VERWENDUNG VON VERNETZTEN KRISTALLEN ALS NEUEM VERFAHREN ZUR IMMOBILISIERUNG VON ENZYMEN

EMPLOI DE CRISTAUX RETICULES COMME NOUVELLE FORME D'IMMOBILISATION D'ENZYMES

(84) Designated Contracting States:
**AT BE CH DE DK ES FR GB GR IT LI LU NL SE**

(30) Priority: **03.08.1990 US 562280**

(43) Date of publication of application:
**14.07.1993 Bulletin 1993/28**

(60) Divisional application:
**98105856.3 / 0 861 888**

(73) Proprietor: **VERTEX PHARMACEUTICALS INCORPORATED**
**Cambridge, MA 02139-4211 (US)**

(72) Inventors:
- **NAVIA, Manuel, A.**
  **Lexington, MA 02173 (US)**
- **ST. CLAIR, Nancy, L.**
  **Charlestown, MA 02139 (US)**

(74) Representative: **VOSSIUS & PARTNER**
**Postfach 86 07 67**
**81634 München (DE)**

(56) References cited:

| | |
|---|---|
| **EP-A- 169 767** | **EP-A- 175 582** |
| **EP-A- 341 503** | **EP-A- 367 302** |
| **WO-A-85/03247** | **US-A- 4 411 996** |

- **WPIL, Derwent Publications Ltd., London (GB); AN 88-061232**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 550 450 B1

## Description

### Background of the Invention

Enzymes are used as industrial catalysts for the large and laboratory scale economical production of fine and specialty chemicals (Jones, J.B., Tetrahedron 42: 3351-3403 (1986)), for the production of foodstuffs (Zaks et. al., Trends in Biotechnology 6: 272-275 (1988)), and as tools for the synthesis of organic compounds (Wong, C.-H., Science 244: 1145-1152 (1989); CHEMTRACTS-Org. Chem. 3: 91-111 (1990); Klibanov, A.M., Acc. Chem. Res. 23: 114-120 (1990)).

Enzyme-based manufacturing can significantly reduce the environmental pollution burden implicit in the large scale manufacturing of otherwise unusable chemical intermediates, as shown in the large scale production of acrylamide using the enzyme, nitrile hydratase (Nagasawa, T. and Yamada; H., Trends in Biotechnology 7: 153-158 (1989)).

Enzymes are also used in biosensor applications to detect various substances of clinical, industrial and other interest (Hall, E., "Biosensors", Open University Press (1990)). In the clinical area, enzymes may be used in extracorporeal therapy, such as hemodialysis and hemofiltration, where the enzymes selectively remove waste and toxic materials from blood (Klein, M. and Langer, R., Trends in Biotechnology 4: 179-185 (1986)). Enzymes are used in these areas because they function efficiently as catalysts for a broad range of reaction types, at modest temperatures, and with substrate specificity and stereoselectivity. Nonetheless, there are disadvantages associated with the use of soluble enzyme catalysts which have limited their use in industrial and laboratory chemical processes (Akiyama et. al., CHEMTECH 627-634 (1988)).

Enzymes are expensive and relatively unstable compared to most industrial and laboratory catalysts, even when they are used in aqueous media where enzymes normally function. Many of the more economically interesting chemical reactions carried out in common practice are incompatible with aqueous media, where, for example, substrates and products are often insoluble or unstable, and where hydrolysis can compete significantly. In addition, the recovery of soluble enzyme catalyst from product and unreacted substrate in the feedstock often requires the application of complicated and expensive separation technology. Finally, enzymes are difficult to store in a manner that retains their activity and functional integrity, for commercially reasonable periods of time (months to years) without having to resort to refrigeration (4°C to -80°C to liquid $N_2$ temperatures), or to maintenance in aqueous solvents of suitable ionic strength, pH, etc.

Enzyme immobilization methods have, in many instances, circumvented these disadvantages. Immobilization can improve the stability of enzyme catalysts and protect their functional integrity in the harsh solvent environments and extreme temperatures characteristic of industrial and laboratory chemical processes (Hartmeier, W., Trends in Biotechnology 3: 149-153 (1985)). Continuous flow processes may be operated with immobilized enzyme particles in columns, for example, where the soluble feedstock passes over the particles and is gradually converted into product. As used herein, the term enzyme immobilization refers to the insolubilization of enzyme catalyst by attachment to, encapsulation of, or by aggregation into macroscopic ($10^{-1}$ mm) particles.

A number of useful reviews of enzyme immobilization methods have appeared in the literature (Maugh, T.H., Science 223: 474-476 (1984); Tramper, J., Trends in Biotechnology 3: 45-50 (1985)). Maugh describes five general approaches to the immobilization of enzymes. These include: adsorption on solid supports (such as ion-exchange resins); covalent attachments to supports (such as ion-exchange resins, porous ceramics or glass beads); entrapment in polymeric gels; encapsulation: and the precipitation of soluble proteins by cross-linking them with bifunc-tional reagents in a random and undefined manner. EP-A-367302 (Soejima et al.) describes a water-soluble cross-linked Achromobacter protease I for use in a process for semi-synthesizing human insulin. EP-A-341503 (Visuri) describes a water-insoluble glucose isomerase which is formed by a crystalline enzyme converted to solid form by cross-linking with dialdehyde (glutaraldehyde) in the presence of a compound containing an amino group. In addition, one can immobilize whole cells (usually dead and made permeable) which have expressed the desired enzyme activity at high levels (e.g., Nagasawa, T. and Yamada, H., Trends in Biotechnology 7: 153-158 (1989)).

Each of these immobilization procedures has its own advantages and limitations and none can be considered optimal or dominating. In most of them, the enzyme catalyst ultimately represents only a small fraction of the total volume of material present in the chemical reactor. As such, the bulk of the immobilized medium is made up of inert, but often costly carrier material. In all of them, the immobilizing interactions of the enzyme catalyst molecules with each other and/or with the carrier material tend to be random and undefined. As a result, although these interactions confer some enhanced stability to the enzyme catalyst molecules, their relative non-specificity and irregularity makes that stabilization sub-optimal and irregular. In most cases, access to the active site of the enzyme catalyst remains ill-defined. In addition, the immobilization methods described above fail to deal with problems associated with storage and refrigeration. Nor can conventionally immobilized enzymes generally be manipulated, as in being exchanged into one or another solvent of choice, without risk to the structural and functional integrity of the enzyme. In practical terms, except for the attached tether to the carrier particle, conventionally immobilized enzymes bear close resemblance to soluble enzymes, and share with them a susceptibility to denaturation and loss of function in harsh environments.

In general, immobilization methods lead to a reduction of observed enzyme-catalyzed reaction rates relative to those obtained in solution. This is mostly a consequence of the limits of inward diffusion of substrate and outward diffusion of product within the immobilized enzyme particle (Quiocho, F.A., and Richards, F.M., Biochemistry 5: 4062-4076 (1967)). The necessary presence of inert carrier in the immobilized enzyme particles increases the mean free path between the solvent exterior of the immobilized enzyme particle and the active site of the enzyme catalyst and thus exacerbates these diffusion problems. When dealing with immobilized cells, the diffusion problem is particularly severe, even if cell walls and membranes are made permeable to substrate and product in some way. One would further be concerned with the multitude of contaminating enzymatic activities, metabolites, and toxins contained in cells, and with the stability of cells in harsh solvents or extreme temperature operating environments. An improved immobilization technique which avoids the limitations of the presently available methods would be helpful in promoting the use of enzymes as industrial catalysts, particularly if it were shown to be useful on a large scale (Daniels, M.J., Methods in Enzymology 136: 371-379 (1987)).

## Summary of the Invention

The present invention relates to a method of immobilizing an enzyme by forming crystals of the enzyme and, generally, also cross linking the resulting crystals through use of a bifunctional reagent; cross-linked immobilized enzyme crystals (referred to as CLECs or CLIECs) made by this method; the lyophilization of CLECs as a means of improving the storage, handling, and manipulation properties of immobilized enzymes and a method of making a desired product by means of a reaction catalyzed by a CLEC or a set of CLECs.

In the method of the present invention, small ($10^{-1}$mm) protein crystals are grown from aqueous solutions, or aqueous solutions containing organic solvents, in which the enzyme catalyst is structurally and functionally stable. In a preferred embodiment, crystals are then cross-linked with a bifunctional reagent, such as glutaraldehyde. This cross-linking results in the stabilization of the crystal lattice contacts between the individual enzyme catalyst molecules constituting the crystal. As a result of this added stabilization, the cross-linked immobilized enzyme crystals can function at elevated temperatures, extremes of pH and in harsh aqueous, organic, or near-anhydrous media, including mixtures of these. That is, a CLEC of the present invention can function in environments incompatible with the functional integrity of the corresponding uncrystallized, uncrosslinked, native enzyme or conventionally immobilized enzyme catalysts.

In addition, CLECs made by this method can be subjected to lyophilization, producing a lyophilized CLEC which can be stored in this lyophilized form at non-refrigerated (room) temperatures for extended periods of time, and which can be easily reconstituted in aqueous, organic, or mixed aqueous-organic solvents of choice, without the formation of amorphous suspensions and with minimal risk of denaturation.

The present invention also relates to CLECs produced by the present method and to their use in laboratory and large scale industrial production of selected materials, such as chiral organic molecules, peptides, carbohydrates, lipids, or other chemical species. Presently, these are typically prepared by conventional chemical methods, which may require harsh conditions (e.g. aqueous, organic or near-anhydrous solvents, mixed aqueous/organic solvents or elevated temperatures) that are incompatible with the functional integrity of uncrystallized, uncrosslinked, native enzyme catalyst. Other macromolecules with catalytic activity can also be incorporated into the proposed CLEC technology. These might include catalytic antibodies (Lerner, R.A., Benkovic, S.J., and Schultz, P.G., Science 252:659-667 (1991)) and catalytic polynucleotides (Cech, T.R., Cell 64:667-669 (1991); Celander, D.W., and Cech, T.R. Science, 251:401-407 (1991)).

The present invention also relates to a method of making a selected product by means of a reaction catalyzed by a CLEC of the present invention.

In an example of the method and practice of the present invention, the enzyme thermolysin, a zinc metalloprotease, was used to synthesize a chiral precursor of the dipeptidyl artificial sweetener, aspartame. The enzyme thermolysin was crystallized from a starting aqueous solution of 45% dimethyl sulfoxide, and 55% 1.4M calcium acetate, 0.05M sodium cacodylate, pH 6.5. The resulting crystals were cross-linked with glutaraldehyde to form a thermolysin CLEC. The thermolysin CLEC was then transferred from the aqueous crystallization solution in which it was made, into a solution of ethyl acetate containing the substrates, N-(benzyloxycarbonyl)-L-aspartic acid (Z-L-Asp) and L-phenylalanine methyl ester (L-Phe-OMe). The thermolysin CLEC was then used to catalyze a condensation reaction of the two substrates to synthesize N-(benzyloxycarbonyl)-L-aspartyl-L-phenylalanine methyl ester (Z-L-Asp-L-Phe-OMe), which is the dipeptidyl precursor of the artificial sweetener aspartame. Using any one of many known techniques (see, e.g. Lindeberg, G., J. Chem Ed. 64: 1062-1064 (1987)) the L-aspartic acid in the synthesized dipeptidyl precursor can be deprotected by the removal of the benzyloxycarbonyl (Z-) group to produce aspartame (L-Asp-L-Phe-OMe).

In a second example of the method and practice of the present invention, the enzyme thermolysin was used to produce thermolysin CLECs. The activity and stability of thermolysin CLECs were compared to that of soluble thermolysin under optimum conditions and conditions of extreme pH and temperature, following incubation in the presence of organic solvents and following incubation in the presence of exogenous protease.

The enzyme thermolysin was crystallized from a solution of 1.2M calcium acetate and 30% dimethyl sulfoxide pH 8.0. The resulting crystals were crosslinked with glutaraldehyde at a concentration of 12.5% to form a thermolysin CLEC. The thermolysin CLEC was then lyophilized by a standard procedure (Cooper, T.G., The Tools of Biochemistry, pp. 379-380 (John Wiley and Sons, NY (1977)) to form a lyophilized enzyme CLEC of thermolysin. This lyophilized CLEC was then transformed directly into the different aqueous, organic, and mixed aqueous/organic solvents of choice without an intervening solvent exchange procedure, without formation of amorphous suspensions, and with minimal risk of denaturation. These solvents included acetonitrile, dioxane, acetone, and tetrahydrofuran, but not to the exclusion of others. Following incubation, activity was assayed spectrophotometrically by cleavage of the dipeptide substrate FAGLA (furylacryloyl-glycyl-L-leucine amide).

In a third example of the method and practice of the present invention, the enzyme elastase (porcine pancreatic) was crystallized from an aqueous solution of 5.5 mg/ml protein in 0.1 M sodium acetate at pH 5.0 at room temperature (Sawyer, L. et al., J. Mol. Biol. 118:137-208). The resulting crystals were crosslinked with glutaraldehyde at a concentration of 5% to form an elastase CLEC. The elastase CLEC was lyophilized as described in Example 2.

In a fourth example of the method and practice of the present invention, and as disclosed here, the enzyme esterase (porcine liver) was crystallized from an aqueous solution of 15 mg/ml protein in 0.25 M calcium acetate at pH 5.6 at room temperature. The resulting crystals were crosslinked with glutaraldehyde at a concentration of 12.5% to form an esterase CLEC. The esterase CLEC was lyophilized as described in Example 2.

In a fifth example of the method and practice of the present invention, and as disclosed here, the enzyme lipase (Geotrichum candidum) was crystallized from an aqueous solution of 20 mg/ml protein in 50 mM Tris at pH 7 at room temperature. The resulting crystals were crosslinked with glutaraldehyde at a concentration of 12.5% to form a lipase CLEC. The lipase CLEC was lyophilized as described in Example 2.

In a sixth example of the method and practice of the present invention, the enzyme lysozyme (hen egg white) was crystallized from an aqueous solution of 40 mg/ml protein in 40 mM sodium acetate buffer containing 5% sodium chloride at pH 7.4 at room temperature (Blake, C.C.F. et al., Nature, 196:1173 (1962)). The resulting crystals were crosslinked with glutaraldehyde at a concentration of 20% to form a lysozyme CLEC. The lysozyme CLEC was lyophilized as described in Example 2.

In a seventh example of the method and practice of the present invention, the enzyme asparaginase (Escherichia coli) was crystallized from an aqueous solution of 25 ng/ml protein in 50 mM sodium acetate and 33% ethanol at pH 5.0 at 4°C. The crystallization is a modification of the procedure described by Grabner et al. [U.S. Patent 3,664,926 (1972)]. As disclosed here, the resulting crystals were crosslinked with glutaraldehyde at a concentration of 7.5% to form an asparaginase CLEC. The asparaginase CLEC was lyophilized as described in Example 2.

Other enzymes which can be immobilized in a similar manner and used to catalyze an appropriate reaction include luciferase and urease. other enzymes, such as those listed in Tables 1-5, can also be crystallized and crosslinked using the present method, to produce a desired CLEC which can, in turn, be used to catalyze a reaction which results in production of a selected product or to catalyze a reaction which is an intermediate step (i.e. one in a series of reactions) in the production of a selected product. It is recognized that although crosslinking helps stabilize the majority of crystals, it is neither necessary nor desirable in all cases. Some crystalline enzymes retain functional and structural integrity in harsh environments even in the absence of crosslinking. Although in the preferred embodiment, the crystal is crosslinked, crosslinking is not always necessary to produce an enzyme crystal useful in the present method.

CLECs have several key characteristics that confer significant advantages over conventional enzyme immobilization methods presently in use. CLECs dispense with the need for a separate, inert support structure. Lack of an inert support will improve substrate and product diffusion properties within CLECs and provides enzyme concentrations within the crystal that are close to the theoretical packing limit for molecules of such size. High enzyme concentrations can lead to significant operational economies through the increased effective activity of a given volume of catalyst, reduction in substrate contact time with enzyme and overall reduction in plant size and capital costs (Daniels, M.J., Methods in Enzymol. 136: 371-379 (1987)). The uniformity across crystal volume and enhanced stability of the constituent enzyme in CLECs creates novel opportunities for the use of enzyme catalysis in harsh conditions, such as elevated temperature, and aqueous, organic or near-anhydrous solvents, as well as mixtures of these. In addition, the restricted solvent access and regular protein environment implicit in a crystal lattice should lead to improved metal ion and co-factor retention for CLECs vs. conventional immobilized enzyme systems.

Brief Description of the Drawings

Figure 1 is a graphic representation of results of assessment of enzymatic activity of soluble and thermolysin CLEC.

Figure 2 is a graphic representation of results of a comparison of pH dependencies of thermolysin CLEC and soluble thermolysin.

Figure 3 is a graphic representation of measurement of the activity of soluble and crystalline thermolysin after incubation at 65°C.

Figure 4 is a graphic representation of results of assessment of resistance of soluble and thermolysin CLEC to exogenous proteolytic degradation.

Figure 5 is a graphic representation of results of the assessment of enzymatic activity for soluble elastase and the corresponding elastase CLEC.

Figure 6 is a graphic representation of the resistance of soluble elastase and the corresponding elastase CLEC to exogenous proteolytic degradation.

Figure 7 is a graphic representation of results of the assessment of enzymatic activity for soluble esterase and the corresponding esterase CLEC.

Figure 8 is a graphic representation of the resistance of soluble esterase and the corresponding esterase CLEC to exogenous proteolytic degradation.

Figure 9 is a graphic representation of results of the assessment of enzymatic activity for soluble lipase and the corresponding lipase CLEC.

Figure 10 is a graphic representation of results of the assessment of enzymatic activity for soluble lysozyme and the corresponding lysozyme CLEC.

Figure 11 is a graphic representation of results of the assessment of enzymatic activity for soluble asparaginase and the corresponding asparaginase CLEC.

Detailed Description of the Invention

A simple, general procedure that assures stability and function for a given enzyme or set of enzymes under conditions which are of interest to the synthetic chemist and which are too harsh for use with enzymes using presently available methods, would be very useful. Cross-linked immobilized enzyme crystals (referred to as CLECs or CLIECs) as described here can be used for this purpose. Stabilization of the crystal lattice and of the constituent enzyme catalysts in the crystal by the cross-linking reaction permits the use of CLECs in environments, including aqueous, organic or near-anhydrous solvents, mixtures of these solvents, extremes of pH and elevated temperatures, which are incompatible with enzyme function using presently available methods. In addition, the stabilization of the crystal lattice in CLECs makes possible the lyophilization of CLECs by standard methods. Lyophilized CLECs can be stored for commercially attractive periods of time (months to years) in the absence of refrigeration, and facilitate the rapid and uncomplicated utilization of CLECs in industrial and laboratory scale processes by the simple addition of solvents of choice, without need for intervening solvent exchange processes. CLECs are also highly resistant to digestion by exogenous proteases. The method of the present invention facilitates the use of versatile enzyme catalysts in mainstream industrial chemical processes, as well as in the laboratory synthesis of novel compounds for research.

Although crosslinking contributes to the stability of a crystal enzyme, it is neither necessary nor desirable in all cases. Some crystallized enzymes retain functional and structural integrity in harsh environments even in the absence of crosslinking. The preferred embodiment of the present method includes cross-linking of a crystal enzyme and is described in detail in the following sections. It is to be understood, however, that crystallized enzymes which are not subsequently cross-linked can be used in some embodiments of the present invention.

The regular interactions between the constituent enzyme molecules in the crystal lattice of a CLEC result in well defined pores of limited size leading to the enzyme molecules within the body of a CLEC. As a result, substrates larger than the available pore size will not penetrate the body of the CLEC particle.

As a consequence of the limited pore size, many enzymatic reactions of commercial and academic interest involving substrates larger than the pore size of the CLECs would be beyond the scope of the present invention. This would include most reactions involving large polymers, such as proteins, polynucleotides, polysaccharides, and other organic polymers, where the number of polymeric subunits would be such as to make the polymer larger than the crystal pore size in CLECs. In such instances, however, catalysis can still take place on the CLEC surface.

The present invention is a method of immobilizing a selected protein, particularly an enzyme, by crystallizing and crosslinking the protein, resulting in production of a crosslinked immobilized enzyme crystal (CLEC) which can be used to catalyze production of a selected product, such as a peptide, carbohydrate, lipid or chiral organic molecule. The present invention further relates to such CLECs and to a method of making a selected product by means of a CLEC-catalyzed reaction or CLEC-catalyzed step in a series of reactions. In one embodiment of the present invention, the dipeptidyl precursor of aspartame has been produced in a condensation reaction catalyzed by cross-linked immobilized thermolysin made by the present method. In another embodiment of this invention, the indicator substrate, FAGLA, has been cleaved to produce a colorimetric product, whose presence is indicative of enzyme activity in a thermolysin CLEC. FAGLA hydrolysis has been used as a model reaction to indicate the robustness of the thermolysin CLEC in a number of environments that would be normally incompatible with that enzyme's activity.

In other embodiments of this invention, the enzymes elastase, esterase, lipase, asparaginase, and lysozyme have been used to cleave various indicated substances, such as p-nitrophenyl acetate (esterase and lipase), succinyl-(ala)3-p-nitroanilide (elastase), 4-methylumbelliferyl N-acetyl-chitrioside (lysozyme) and NADH (asparaginase).

By the method of this invention, one of ordinary skill in the art can adapt a protocol for making a desired product by means of a reaction catalyzed by an immobilized enzyme. The enzyme of interest, when crystallized from an appropriate solution, can be cross-linked with glutaraldehyde or other suitable bifunctional reagent in the crystallization solution to produce a CLEC of that enzyme. Subsequently, the CLEC of the enzyme of choice can be lyophilized as described in Example 2.

There are several advantages which the use *of a* CLEC offers over presently-available enzyme-catalyzed methods. For example, the cross-linked crystal matrix in a CLEC provides its own support. Expensive carrier beads, glasses, gels, or films are not required in order to tie down the enzyme catalyst, as they are in presently-available immobilization methods. As a result, the concentration of enzyme in a CLEC is close to the theoretical packing limit that can be achieved for molecules of a given size, greatly exceeding densities achievable even in concentrated solutions. The entire CLEC consists of active enzyme (and not inactive carrier), and thus, the diffusion-related reduction of enzyme reaction rates usually observed with conventionally immobilized enzymes relative to enzymes in solution should be minimized, since the mean free path for substrate and product between active enzyme and free solvent will be greatly shortened for CLECs (compared to a conventional immobilized enzyme carrier particles). These high protein densities will be particularly useful in biosensor, analytical and other applications requiring large amounts of protein in small volumes. In industrial processes, the superior performance and compactness of CLECs results in significant operating economies, by increasing the effective activity of a given volume of catalyst, thereby allowing reductions in plant size, as well as capital costs (Daniels, M.J., Methods in Enzymol. 136: 371-379 (1987)). CLECs are relatively monodisperse, with a macroscopic size and shape reflecting natural crystal growth characteristics of the individual enzyme catalysts. Replacement of existing carrier-immobilized enzyme media with CLECs should not be difficult, since both systems are comparable in size and shape, and both can be similarly recovered from feedstock by any number of simple methods, including basic economical operations such as filtration, centrifugation, decantation of solvent, and others.

In addition, the use of lyophilized CLECs permits routine handling and storage of these materials prior to use (dry storage at room temperature without refrigeration, for extended periods of time). Lyophilized CLECs also allow for routine formulation by direct addition of solvents and substrates of interest, without lengthy solvent exchange processes, or the formation of amorphous suspensions. The lyophilized CLEC form extends the general utility of the enzymes as catalysts to a broader spectrum of enzymes and functional conditions.

A second advantage of a CLEC is that cross-linking of the crystallized enzyme stabilizes and strengthens the crystal lattice and the constituent enzyme molecules, both mechanically and chemically. As a result, a CLEC may be the only means of achieving significant concentrations of active enzyme catalyst in harsh aqueous, organic, near-anhydrous solvents, or in aqueous-organic solvent mixtures. The use of enzymes as catalysts in organic syntheses has been hampered by their tendency to denature in the presence of non-aqueous solvents, and particularly, in mixtures of aqueous and non-aqueous solvents (Klibanov, A.M., Trends in Biochemical Sciences, 14:141-144 (1989)). In CLECs, the restriction of conformational mobility that leads to stability is provided by the inter- molecular contacts and cross-links between the constituent enzyme molecules making up the crystal lattice, rather than by the near-absence of water in the medium. As a result, intermediate water concentrations can be tolerated by enzymes when formulated as CLECs, as has previously not been possible (see Table 12). In commercial applications, aqueous-organic solvent mixtures allow manipulation of product formation by taking advantage of relative solubilities of products and substrates. Even in aqueous media, enzyme catalysts, immobilized or soluble, are subject to mechanical forces within a chemical reactor that can lead to denaturation and a shortened half-life. The chemical cross-links within the CLEC provide the necessary mechanical strength (Quiocho and Richards, Proc. Natl. Acad. Sci. (USA) 52: 833-839 (1964)) that results in increased reactor life for the enzyme catalyst.

A third advantage of a CLEC is that as a result of its crystalline nature, a CLEC can achieve uniformity across the entire cross-linked crystal volume. Crystalline enzymes as described herein are grown and cross-linked in an aqueous environment and, therefore, the arrangement of molecules within the crystal lattice remains uniform and regular. This uniformity is maintained by the intermolecular contacts and chemical cross-links between the enzyme molecules constituting the crystal lattice, even when exchanged into other aqueous, organic or near-anhydrous media, or mixed aqueous/organic solvents. In all of these solvents, the enzyme molecules maintain a uniform distance from each other, forming well-defined stable pores within the CLECs that facilitate access of substrate to the enzyme catalysts, as well as removal of product. Uniformity of enzyme activity is critical in industrial, medical and analytical applications where reproducibility and consistency are paramount.

A fourth advantage of using a CLEC is that it should exhibit an increased operational and storage half-life. Lattice interactions, even in the absence of cross-linking, are known to stabilize proteins, due in part to restrictions of the conformational degrees of freedom needed for protein denaturation. In CLECs, the lattice interactions, when fixed by chemical cross-links, are particularly important in preventing denaturation, especially in mixtures of aqueous and non-aqueous solvents (Klibanov, A.M., Trends in Biochemical Sciences 14: 141-144 (1989)). Enzymes that have been in the crystalline state for months or years routinely retain a high percentage of their catalytic activity. Cross-linked immobilized enzyme crystals stored in anhydrous solvents will be even further protected from microbial contamination and damage, which is a serious problem in storing large quantities of protein in a nutrient rich, aqueous environment. In the case of a lyophilized CLEC, the immobilized enzyme is stored in the absence of solvent. That, and the stabilization achieved by cross-linking allows for the storage in the absence of refrigeration for long periods of time.

A fifth advantage of using a CLEC is that it should exhibit enhanced temperature stability as a consequence of the cross-links stabilizing the crystal lattice. Carrying out reactions at a higher temperature than that used with conventional methods would increase reaction rates for the chemical reactions of interest, both thermodynamically, and by enhancing the diffusion rate into and out of the crystal lattice of CLECs. These combined effects would represent a major improvement in reaction efficiency, because they would maximize the productivity of a given quantity of enzyme catalyst, which is generally the most expensive component of the reaction process (Daniels, M.J., Methods in Enzymol. 136: 371-379 (1987)). The temperature stability exhibited by CLECs is remarkable because most enzyme systems require mild reaction conditions. CLECs would also be stabilized against denaturation by transient high temperatures during storage.

A final advantage of use of a CLEC is that pores of regular size and shape are created between individual enzyme molecules in the underlying crystal lattice. This restricted solvent accessibility greatly enhances the metal ion or cofactor retention characteristics of CLEC as compared to conventionally immobilized enzymes and enzymes in solution. This property of CLEC will permit the use of economically superior continuous-flow processes in situations (see e.g. Oyama et. al. Methods in Enzymol. 136 503-516 (1987)) where enzyme would otherwise be inactivated by metal ion or cofactor leaching. For example, in the thermolysin-mediated synthesis of the dipeptidyl aspartame precursor, Z-L-Asp-L-Phe-OMe, conventionally immobilized enzyme is known to lose catalytic activity in continuous-flow column processes, in part through the leaching of calcium ions essential for thermolysin activity. In practice, leaching of calcium ions has forced the use of less efficient batch processes (Nakanishi et. al., Biotechnology 3: 459-464 (1985)). Leaching occurs when calcium ion complexes are formed with substrate Z-L-Asp, in competition with the natural calcium binding sites on the surface of the enzyme, resulting in the loss of catalytic activity. The high density of enzyme, and the correspondingly limited volume accessible to solvent in the interstices of the CLECs, discourages the formation of the competing L-Asp-$Ca^{++}$ complexes responsible for metal ion leaching.

Preparation of CLECs - enzyme crystallization

In the method of the present invention, a cross-linked immobilized enzyme crystal (or CLEC) is prepared as follows:

Enzyme crystals are grown by the controlled precipitation of protein out of aqueous solution, or aqueous solution containing organic solvents. Conditions to be controlled include, for example, the rate of evaporation of solvent, the presence of appropriate co-solutes and buffers, and the pH and temperature. A comprehensive review of the various factors affecting the crystallization of proteins has been published by McPherson (Methods Enzymol. 114: 112 (1985)). In addition, both McPherson and Gilliland (J. Crystal Growth 90: 51-59 (1988)) have compiled comprehensive lists of all proteins and nucleic acids that have been reported as crystallized, as well as the conditions that lead to their crystallization. A compendium of crystals and crystallization recipes, as well as a repository of coordinates of solved protein and nucleic acid crystal structures, is maintained by the Protein Data Bank (Bernstein et. al. J. Mol. Biol. 112: 535-542 (1977)) at the Brookhaven National Laboratory. Such references can be used to determine the conditions necessary for the crystallization of a given protein or enzyme previously crystallized, as a prelude to the formation of an appropriate CLEC, and can guide the formulation of a crystallization strategy for proteins that have not. Alternatively, an intelligent trial and error search strategy (see eg., Carter, C.W. Jr. and Carter, C.W., J. Biol. Chem. 254: 12219-12223 (1979)) can, in most instances, produce suitable crystallization conditions for most proteins, including, but not limited to, those discussed above, provided that an acceptable level of purity can been achieved for these. The level of purity required can vary widely from protein to protein. In the case of lysozyme, for example, the enzyme has been crystallized directly from its unpurified source, the hen egg-white (Gilliland, G.L., J. Crystal Growth 90: 51-59 (1988)).

For use as CLECs in the method of this invention, the large single crystals which are needed for X-ray diffraction analysis are not required, and may, in fact, be undesirable because of diffusion problems related to crystal size. Microcrystalline showers (ie. crystals of order $10^{-1}$ mm in size/cross section) are suitable for CLECs and are often observed, though seldom reported in the X-ray crystallographic literature. Micro-crystals are very useful in the method of this invention to minimize problems with diffusion (see eg., Quiocho, F.A., and Richards, F.M., Biochemistry 5: 4062-4076 (1967)).

In general, crystals are produced by combining the protein to be crystallized with an appropriate aqueous solvent or aqueous solvent containing appropriate precipitating agents, such as salts or organics. The solvent is combined with the protein at a temperature determined experimentally to be appropriate for the induction of crystallization and acceptable for the maintenance of protein stability and activity. The solvent can optionally include co-solutes, such as divalent cations, co-factors or chaotropes, as well as buffer species to control pH. The need for co-solutes and their concentrations are determined experimentally to facilitate crystallization. In an industrial scale process, the controlled precipitation leading to crystallization can best be carried out by the simple combination of protein, precipitant, co-solutes, and optionally buffers in a batch process. Alternative laboratory crystallization methods, such as dialysis, or vapor diffusion can also be adapted. McPherson (Methods Enzymol. 114: 112 (1985)), and Gilliland (J. Crystal Growth 90: 51-59 (1988)) include a comprehensive list of suitable conditions in their reviews of the crystallization literature. Occasionally, incompatibility between the cross-linking reagent and the crystallization medium might require exchanging the crystals into a more suitable solvent system.

Many of the proteins for which crystallization conditions have already been described in the literature, have considerable potential as practical enzyme catalysts in industrial and laboratory chemical processes, and are directly subject to formulation as CLECs within the method of this invention. Table 1 is a sampling of enzymes that have.already been crystallized. Note that the conditions reported in most of these references have been optimized for the growth of large, diffraction quality crystals, often at great effort. Some degree of adjustment of conditions for the smaller crystals used in making CLECS might be necessary in some cases.

Table 1

| Enzyme | Microbial or biological source | References (including those cited therein) |
|---|---|---|
| • alcohol dehydrogenase | horse liver | Eklund *et al., J.Mol.Biol.* **146**: 561-587 (1981) |
| • alcohol oxidase | *Pichia pastoris* | Boys *et al., J.Mol.Biol.* **208**: 211-212 (1989)<br>Tykarska *et al., J.Protein Chem.* **9**: 83-86 (1990) |
| • aldolase (fructose-bisphosphate) | rabbit muscle | Eagles *et al., J.Mol.Biol.* **45**: 533-544 (1969)<br>Heidmer *et al., Science* **171:** 677-680 (1971) |
| | calf muscle | Goryunov *et al., Biofizika* **14**: 1116-1117(1969) |
| | human muscle | Millar *et al., Trans.Roy.Soc.Lond.* **B293**: 209-214 (1981) |
| | *Drosophila melanogaster* | Brenner *et al., J..Biol.Chem.* **257**: 11747-11749 (1982) |
| • aldolase (PKDG) | *Pseudomonas putida* | Vandlen *et al., J.Biol.Chem.* **248**: 2251-2253 (1973) |
| • alkaline phosphatase | *Escherichia coli* | Sowadski *et al., J.Mol.Biol.* **150**: 245-272 (1981) |
| • asparaginase | *Erwinia carotova* | North *et al., Nature* **224**: 594-595 (1969) |
| | *Escherichia coli* | Epp *et al., Eur.J.Biochem.* **20**: 432-437 (1971) |
| | *Escherichia coli* | Yonei *et al., J.Mol.Biol.* **110:** 179-186(1977) |
| | *Proteus vulgaris* | Tetsuya *et al., J.Biol.Chem.* **248**: 7620-7621 (1972) |
| • carbonic anhydrase | human erythrocyte (C) | Kannan *et al., J.Mol.Biol.* **12**: 740-760 (1965) |
| | human erythrocyte (B) | Kannan *et al., J.Mol.Biol.* **63:** 601-604 (1972) |
| | bovine erythrocyte | Carlsson *et al. J.Mol.Biol.* **80**: 373-375 (1973) |
| • catalase | horse erythrocyte | Glauser *et al., Acta Cryst.* **21**: 175-177(1966) |
| | *Micrococcus luteus* | Marie *et al., J.Mol.Biol.* **129:** 675-676 (1979) |
| | *Penicillium vitale* | Vainshtein *et al., Acta Cryst.* **A37:** C29 (1981) |
| | bovine liver | Eventoff *et. al., J.Mol.Biol.* **103:** 799-801 (1976) |
| • creatine kinase | bovine heart | Gilliland *et al., J.Mol.Biol.* **170:** 791-793 (1983) |
| | rabbit muscle | McPherson, *J.Mol.Biol.* **81**: 83-86 (1973) |

Table 1 (continued)

| Enzyme | Microbial or biological source | References (including those cited therein) |
|---|---|---|
| • glutaminase | *Actenobacter glutanimasificans*<br>*Pseudomonas 7A* | Wlodawer *et al., J.Mol.Biol.* **99:** 295-299 (1975)<br>Wlodawer *et al., J.Mol.Biol.* **112:** 515-519 (1977) |
| • glucose oxidase | *Aspergillus niger* | Kalisz *et al., J.Mol.Biol.* **213:** 207. 209 (1990) |
| • β-lactamases | *Staphylococcus aureus*<br>*Bacillus cereus* | Moult *et al., Biochem* ***J.*** **225:** 167-176 (1985)<br>Sutton *et al., Biochem* ***J.*** **248:** 181-188 (1987) |
| • lacate dehydrogenase | porcine<br>chicken<br>dogfish<br>*Bacillus stearothermophilus* | Hackert *et al., J.Mol.Biol.* **78:** 665-673 (1973)<br>Pickles *et al., J.Mol.Biol.* ***9:*** 598-600 (1964)<br>Adams *et al., J.Mol.Biol.* **41:** 159-188 (1969)<br>Schar *et al., J.MolBiol.* **154:** 349-353 (1982) |
| • lipase | *Geotrichum candidum*<br>horse pancreatic<br><br>*Mucor meihei*<br>human pancreatic | Hata *et al., J.Biochem.* **86:** 1821-1827 (1979)<br>Lombardo. *et al.,J.Mol.Biol.* **205:** 259-261 (1989)<br>Brady *et al.,Nature* **343:** 767-770 (1990)<br>Winkler *et al., Nature* **343:** 771-774 (1990) |
| • luciferase | *Firefly* | Green,A,A.,et *al., Biochem. Biophys. Acta.* **20:** 170 (1956) |
| • luciferase | *Vibrio harveyii* | Swanson *et al ,J.Biol.Chem.* **260:** 1287-1289 (1985) |
| • nitrile hydratase | *Brevibacterium R312*<br><br>*P. chlororaphis B23* | Nagasawa *et al, Biochem.Biophys.Res. Commun.* **139:** 1305-1312 (1986)<br>Nagasawa *et.al., Eur.J.Biochem.* **162:** 691-698 (1987) |
| • peroxidase | horseradish<br><br>horseradish roots (Type E4)<br>Japanese radish | Braithwaite *et al., J.Mol.Biol.* **106**: 229-230 (1976)<br>Aibara *et al., J.Biochem.* **90:** 489-496 (1981)<br>Morita, *Acta Cryst.* **A28:** S52 (1979) |
| • peroxidase (chloride) | *Caldaromyces fumago* | Rubin *et al., J.Biol.Chem.* **257:** 7768-7769 (1982) |
| • peroxidase (cytochrome) | *Sarchomyces cerevisae* | Poulos *et al., J.Biol.Chem.* **253:** 3730-3735 (1978) |
| • peroxidase (glutathione) | bovine erythrocyte | Ladenstein *et al.J.Mol.Biol.* **104:** 877-882 (1979) |
| • subtilisin | *Bacillus subtilis* (Novo)<br>*Bacillus amyloliquefaciens* (BPN')<br>*Bacillus subtilis* (Carlsberg) | Drenth *et al., J.Mol.Biol.* **28:** 543-544 (1967)<br>Wright *et al., Nature* **221:** 235-242 (1969)<br>Petsko *et al., J.Mol.Biol.* **106:** 453-456 (1976) |
| • superoxide dismutase | bovine<br><br>spinach<br>*Saccharomyces cerevisiae, Escherichia coli*<br>*Bacillus stearothermophillus*<br>*Pseudomonas ovalis* | Richardson *et al., J.Biol.Chem.* **247:** 6368-6369 (1972)<br>Morita *et al., J.Mol.Biol.* 86: 685-686 (1974)<br>Beem *et al., J.Mol.Biol.* 105: 327-332 (1976)<br><br>Bridgen *et al., J.Mol.Biol.* 105: 333-335 (1976)<br>Yamakura *et al., J.Biol.Chem.* **251:** 4792-4793 (1976) |
| • thermolysin | *Bacillus thermoproteolyticus* | Matthews *et al., Nature New Biol.* **238**: 37-41 (1972) |
| • urease | jack bean | Sumner,J.B., *J.Biol. Chem.* **69**: *435* (1926) |

Table 1 (continued)

| Enzyme | Microbial or biological source | References (including those cited therein) |
|---|---|---|
| • xylose isomerase | *Streptomyces rubiginosus* | Carrell *et al., J.Biol.Chem.* **259**: 3230-3236 (1984) |
| | *Arthrobacter B3728* | Akins *et al.*, *Biochym.Biophys Acta* **874**: 375-377 (1986) |
| | *Streptomyces oilvochromogenes* | Farber *et al.*, *Protein Engineering* **1**: 459-466 (1987) |
| | *Streptomyces violaceoniger* | Glasfeld *et al., J.Biol.Chem.* **263**: 14612-14613 (1988) |
| | *Actinoplanes missouriensis* | Rey *et al., Proteins:Struc.Func.Genet* **4:** 165-172 (1988) |

Preparation of CLECs - cross-linking reaction

Once crystals are grown in a suitable medium, they can be cross-linked. Cross-linking results in stabilization of the crystal lattice by introducing covalent links between the constituent enzyme molecules in the crystal. This makes possible the transfer of enzyme into an alternate reaction environment that might otherwise be incompatible with the existence of the crystal lattice, or even with the existence of intact undenatured protein. Cross-linking can be achieved by a wide variety of bifunctional reagents, although in practice, simple, inexpensive glutaraldehyde has become the reagent of choice. (For a representative listing of other available cross-linking reagents, one can consult, for example, the 1990 catalog of the Pierce Chemical Company).

Cross-linking with glutaraldehyde forms strong covalent bonds between primarily lysine amino acid residues within and between the enzyme molecules in the crystal lattice that constitute the crystal.. The cross-linking interactions prevent the constituent enzyme molecules in the crystal from going back into solution, effectively insolubilizing or immobilizing the enzyme molecules into microcrystalline (ideally $10^{-1}$ mm) particles. The macroscopic, immobilized, insolubilized crystals can then be readily separated from the feedstock containing product and unreacted substrate by simple procedures such as filtration, decantation, and others. They can also be used in CLEC packed columns in continuous flow processes, where they exhibit enhanced cofactor and metal ion retention properties.

By the method of this invention, CLECs are obtained for use as enzyme catalysts in existing and novel environments. The enhanced stability of the CLECs, which results from the cross-linking reaction, makes it possible to transfer the CLEC into a solvent (e.g., aqueous, organic or near-anhydrous solvents, or a mixture of these), in which it would otherwise be incompatible, and to carry out chemical reactor operation at elevated temperatures of extremes of pH. The macroscopic CLEC catalyst particles can also be readily manipulated, allowing recovery from feedstock by simple methods, such as filtration, centrifugation, or decantation of solvent. In addition, these can be used in packed columns in continuous flow processes.

Preparation of CLECs - lyophilization

A suspension of one volume of cross-linked thermolysin crystals in ten volumes of demineralized water at pH 7.0 was lyophilized overnight using a VirTis Model #24 lyophilizer. Lyophilized crystals were stored at room temperature or at 4°C prior to reconstitution, which was accomplished by adding ten volumes of the solvent of choice directly onto crystals taken from storage. Re-hydrated crystals were reconstituted in 10mM calcium acetate buffer at pH 7.0 for the FAGLA cleavage experiments. Reconstituted lyophilized CLECs were routinely stored at room temperature. In contrast, soluble enzyme required storage at -70°C to maintain specific activity longer than a week. This protocol was used for all the enzymes described in the exemplification included here.

Synthesis of aspartame precursor with thermolysin CLEC

The method of the present invention, by which cross-linked crystal enzymes are produced, is described below and exemplified by the production of cross-linked immobilized enzyme crystals of thermolysin for use in the production of the dipeptidyl precursor of aspartame, in ethyl acetate, which is a near-anhydrous organic, solvent. Thermolysin, a protein which has been crystallized and whose structure has been solved at 1.6Å resolution (Holmes and Matthews, J. Mol. Biol. 160: 623-639 (1982)), is one example of an enzyme which can be used as a CLEC in the present method. Thermolysin is used in the manufacture of the artificial sweetener aspartame (Isowa et. al. U.S. Patent #4,436,925 (1984); Lindeberg, J. Chem. Ed. 64: 1062-1064 (1987); Nakanishi et. al., Biotechnology 3: 459-464 (1985); oyama, et.

al., Methods in Enzymol. 136: 503-516 (1987)). At the present time, most aspartame appears to be produced by a conventional synthetic chemistry approach, although use of conventionally immobilized thermolysin in near-anhydrous media has produced encouraging results (Oyama et. al., J. Org. Chem. 46: 5242-5244 (1981); Nakanishi et. al., Biotechnology 3: 459-464 (1985)). Improvement in the enzymatic approach to aspartame production, such as is possible through use of the present method, would make it competitive with the presently-used method, both in terms of convenience and cost (Oyama, et. al., Methods in Enzymol. **136**: 503-516 (1987)).

Assessment of thermolysin CLECs

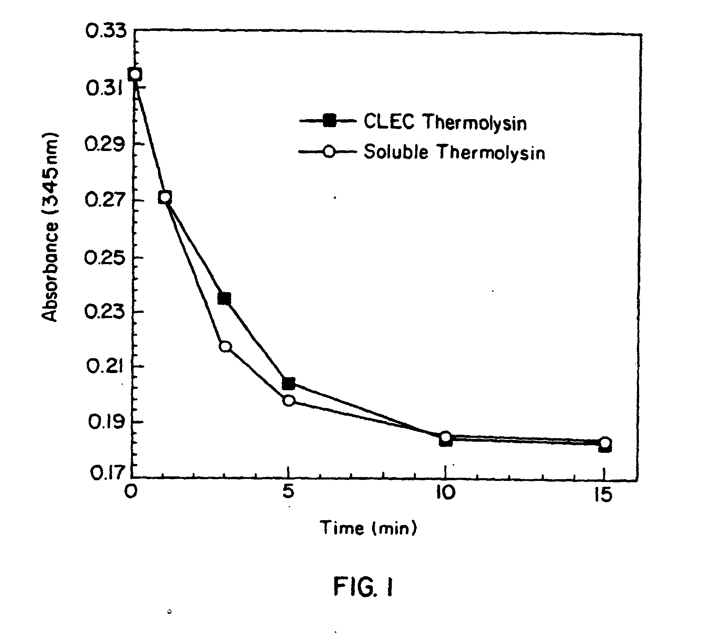

The method of the present invention has also been used to produce thermolysin CLECs which have been assessed as to their pH dependence and stability, stability at elevated temperature, resistance to exogenous proteolysis and stability in the presence of an organic solvent. Thermolysin CLECs were compared to soluble thermolysin, as described in detail in Example 2 and Figures 1-4. Results of the assessment showed the following:

1. As to pH dependence and stability, both forms demonstrate maximum activity at pH 7 and demonstrate similar activity in the acidic range. In the alkaline pH range, the CLEC maintains maximum activity to pH 10; the soluble thermolysin has 75% activity at pH 8.5, only 25% activity at pH 9 and is completely inactive at pH 9.5.
2. The additional stabilization achieved in CLECs results in enzymatic activity at higher temperatures than is possible with soluble thermolysin. Enhanced stability of CLEC thermolysin at lower temperatures makes storage simpler than it is for the soluble enzyme. Thermal stability and resistance to autolysis was also demonstrated for thermolysin CLECs, which retained maximum activity after five days of incubation at 65°C. In contrast, soluble thermolysin lost 50% of its initial activity after two hours incubation and demonstrated negligible activity after 24 hours incubation at 65°C.
3. Enzymatic activity of thermolysin CLECs was unaffected by four days' incubation in the presence of the powerful streptococcal protease, Pronase®. In contrast, soluble thermolysin was rapidly degraded and lost all activity after 90 minutes incubation.
4. Thermolysin CLECs and soluble thermolysin exhibited markedly different stability in the presence of organic solvents, as shown in Table 12. The thermolysin CLECs retained greater than 95% maximum activity following incubation with all organic solvents assessed.

These features of thermolysin CLECs and other enzyme CLECs make them particularly useful, since they are easier to store, more stable and less easily inactivated or degraded than corresponding soluble enzymes.

Assessment of Elastase CLECs

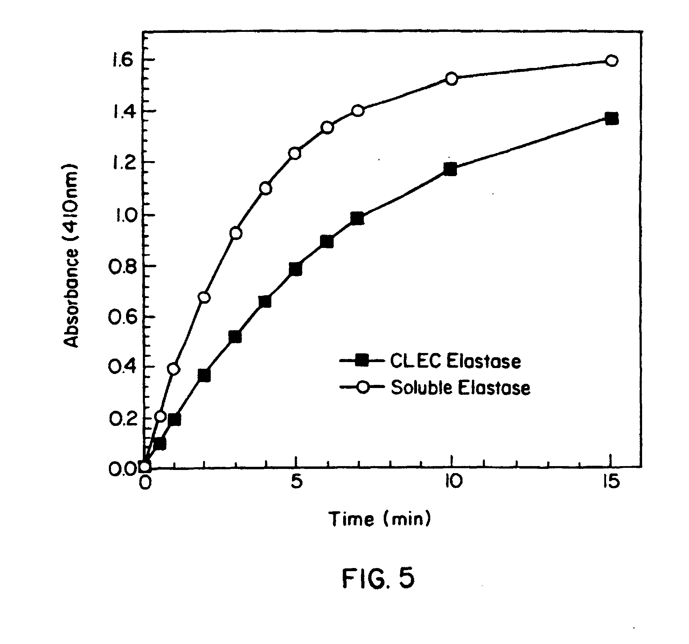

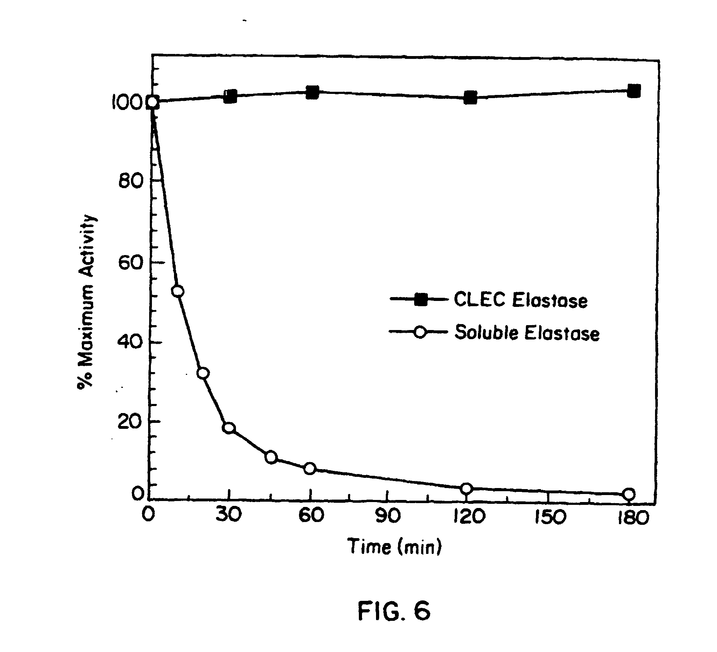

The method of the present invention has also been used to produce elastase CLECs which have been assessed as to their activity and resistance to exogenous proteolysis. Elastase CLECs were compared to soluble elastase, as described in detail in Example 3 and Figures 5 and 6. Results of the assessment demonstrated the following:

1. Elastase CLECs retain approximately 50% activity compared to soluble enzyme.
2. Soluble elastase was rapidly degraded by protease. Activity of soluble elastase was reduced to 50% of initial activity following ten minutes incubation in the presence of protease. After one hour incubation the soluble enzyme had lost more than 90% of its initial activity. In contrast the enzymatic activity of the elastase CLEC was unaffected by incubation with protease.

Assessment of Esterase CLECs

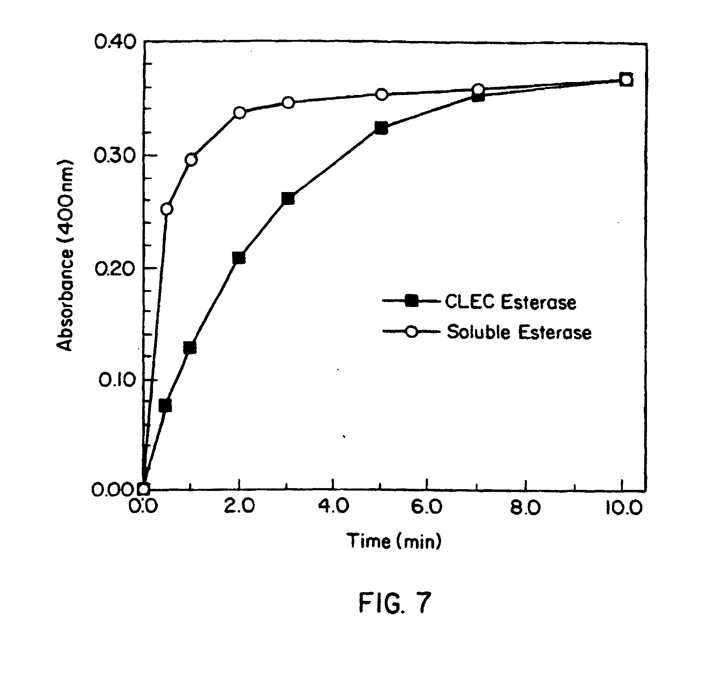

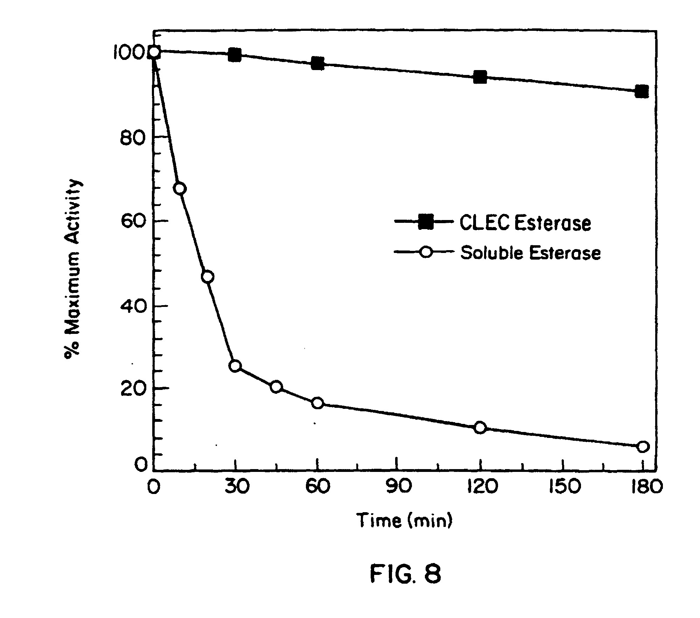

The method of the present invention has also been used to produce esterase CLECs which have been assessed as to their activity and resistance to exogenous proteolysis. Esterase CLECs were compared to soluble esterase, as described in detail in Example 4 and Figures 7 and 8. Results of the assessment demonstrated the following:

1. Esterase CLECs retain approximately 50% activity compared to soluble enzyme.
2. Soluble esterase was highly susceptible to proteolytic degradation. Activity of soluble esterase was reduced to 50% of initial activity following ten minutes incubation in the presence of protease. After one hour incubation the soluble enzyme had lost more than 90% of its initial activity. In contrast the enzymatic activity of the esterase CLEC was unaffected by incubation with protease.

Assessment of Lipase CLECs

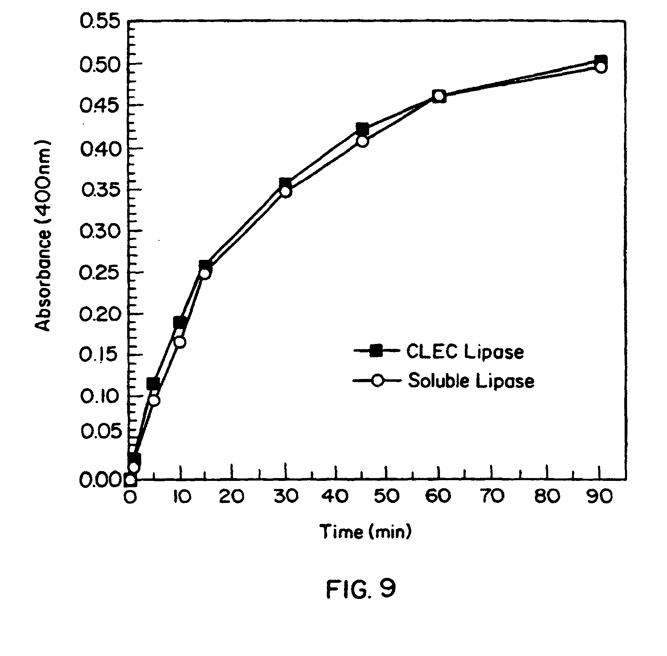

The method of the present invention has also been used to produce lipase CLECs which have been assessed as to their activity. Lipase CLECs were compared to soluble lipase, as described in detail in Example 5 and Figure 9. Results of the assessment demonstrated that lipase CLECs retain approximately 90% activity compared to soluble enzyme.

Assessment of Lysozyme CLECs

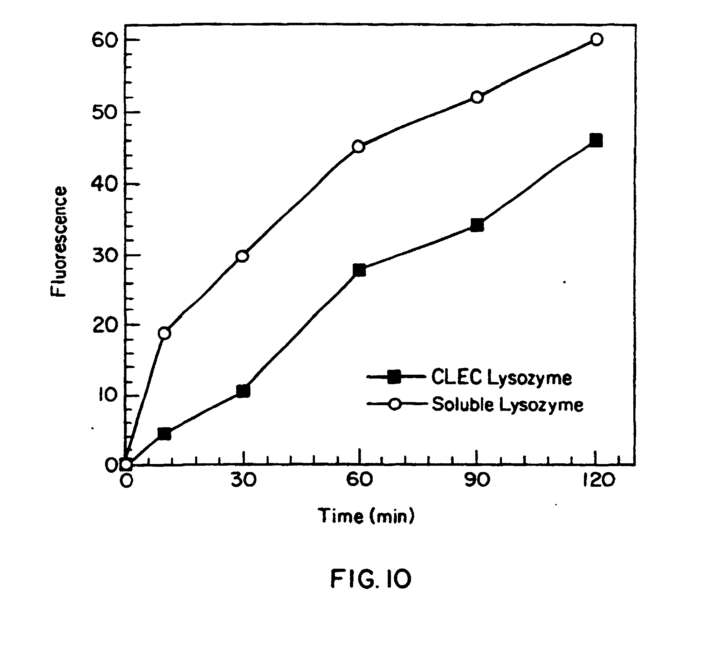

The method of the present invention has also been used to produce lysozyme CLECs which have been assessed as to their activity and resistance to exogenous proteolysis. Lysozyme CLECs were compared to soluble lysozyme as described in detail in Example 6 and Figure 10. Results of the assessment demonstrated that lysozyme CLECs retain approximately 50% activity compared to soluble enzyme.

Assessment of Asparaginase CLECs

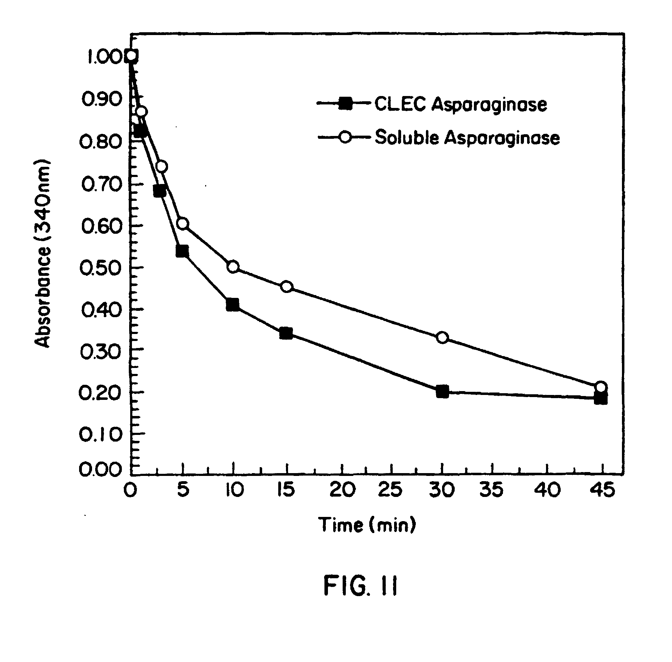

The method of the present invention has also been used to produce asparaginase CLECs which have been assessed as to their activity. Asparaginase CLECs were compared to soluble asparaginase, as described in detail in Example 7 and Figure 11. Results of the assessment demonstrated the following asparaginase CLECs retain approximately 77% activity compared to soluble enzyme.

General applicability of CLECs

As disclosed here, CLECs represent a novel technology with broad use in many areas, including, but not limited to, industrial scale syntheses, laboratory tools, biosensors, and medical applications. Examples of various systems using conventionally immobilized enzyme methods in their execution are given in Tables 2-5 below. One skilled in the art should be able to adapt these, and similar systems, to the CLEC technology disclosed in this application. To illustrate this, specific examples are discussed in more detail from each of the categories listed.

Table 2 below lists examples which use conventionally immobilized enzymes in an industrial process, which examples can be readily adapted to the CLEC technology disclosed here.

Table 2

| Enzyme | Production or application | Substrates | References (including those cited) |
|---|---|---|---|
| • thermolysin | • aspartame precursor | Z-Asp, L-Phe-OMe | Oyama *et al., J. Org. Chem.* **46:** 5242-5244 (1981)<br>Nakanishi *et al., Trends in Biotechnology* **3:** 459-464 (1985) |
| • subtilisin | • aspartame | L-Asp-L-Phe, OMe | Davino,A.A., US Patent 4293648 (1981) |
| • lipase | • cocoa fat substitutes | palm oils | Harwood, J., *Trends in Biochemical Sciences* **14:** 125-126 (1989)<br>Macrae, A.R., *J. Am. Oil Chem Soc*, **60:** 291-294 (1983) |
| • nitrile hydratase, nitrilases, amidase | • acrylamide | acrylonitrile | Nagasawa, T. and Yamada, H., *Trends in Biotechnology* **7:** 153-158 (1989) |

Table 2 (continued)

| Enzyme | Production or application | Substrates | References (including those cited) |
|---|---|---|---|
| • amino acylase (fungal) | • amino acid | N-acyl-D,L amino acids | Schmidi-Kastner,G. & Egerer, P. in *Biotechnology* **vol 6a:** 387-421 (1984) and references therein. |
| • amino acid esterase, | resolution | esters of D,L amino acids | |
| • subtilisin | | | |
| • amidases | | amides of D,L amino acids | |
| • hydantionases | | | |
| • specific dehydrogenases | | hydantoins | Fusee,M.C., *Methods in Enzymology* **136:** 463 (1987) |
| • amino peptidase | | a-hydroxycarboxylic acids | |
| • transaminase | | | |
| | | | Fusee,M.C., *Methods in Enzymology* **136:** 463 (1987) |
| • amino acid dehydrogenase + formate dehydrogenase | • amino acid production: general | keto or hydroxy acids | Rozzell, J.D., *Methods in Enzymology* **136:** 479 (1987) |
| • L-aspartase | • amino acid production: specific L aspartic acid | fumarate/fumaric acid | Enzymes in Industry; Ed Gerhartz.W.,VCH Press 1990 |
| • L-aspartate 4-decarboxylase | L-alanine | L-aspartic acid, ammonium fumarate | |
| aspartase + L aspartate 4 decar-boxylase | L-lysine | D,L-a amino e-caprolactam (ACL) | |
| • ACL hydrolase | | | |
| • L-ACT hydrolase | L-cysteine | DL-2amino2 thiazoline 4carboxylic acid | |
| | L-isoleucine | | |
| | L-methionine | | |
| • lyase | L-phenylalanine | cinnamate | |
| • L-tryptophan synthetase | L-tryptophan | indole, L-serine | |
| | L-valine | | |
| • fumarase | L-malic acid | fumarate | |
| • hydantoinase | D n carbamoyl p-hydroxy-phenyl glycine | 5p-hydroxy hydantoin | |
| • lipases, esterases | • resolution of racemates by stereoselective synthesis | synthetic chemistry | Jones, J.B., *Tetrahedron* **42:** 3351-3403 (1988) Butt, S. and Roberts, S.M., *Natural Product Reports* 489-503 (1986), and references cited therein for a more comprehensive review of this area |
| • fumarase | • L-malic acid | fumaric acid | Chibata *et al*., *Methods in Enzymology* **136:** 455 (1987) |

Table 2 (continued)

| Enzyme | Production or application | Substrates | References (including those cited) |
|---|---|---|---|
| • lactase, β-galactosidases | • disaccharide synthesis eg galactosyl-N- acetyl galactosamine | lactose & N-acetyl galactosamine | Larsson *et al., Methods in Enzymology* **136:** 230 (1987) |
| • lipase, esterase | • L-menthol | 4 isomer mix | Fukui,S., Tanaka,A., *Methods in Enzymology* **136:** 293 (1987) |
| • amidases | • D-valine (intermediate for pyrethroid insecticide fluvinate) | D.L. amino acid amide | Schmidt-Kastner,G. & Egerer, P. in *Biotechnology* vol **6a:** 387-421 (1984) and references therein. |
| • lipase (Candida cylindricea) | • R(+)2 phenoxy-propionic acids (herbicides) | 2 chloro propionic acids | *Biocatalysts in Organic Syntheses* eds Tramper, van de Plas & Linko; Proceedings of International Symposium in Netherlands 1985 |
| • lipases, esterases, amidases, aldolases<br>• proteases, peptidases<br>• yeast lipase | • organic syntheses monoglycerides peptides<br>• 2(p-chlorophenoxy) propionic acid: herbicide | resolution of racemic ester | Jones, J.B., *Tetrahedron* **42:** 3351-3403 (1988) Butt, S. and Roberts, S.M., *Natural Product Reports* 489-503 (1986), and references cited therein for a more comprehensive review of this area |
| • strictodine synthetase | • alkaloid production eg strictosidine | | Pfitzner *et al., Methods in Enzymology* **136:** 342 (1987) |
| • penicillin acylase • penicillin amidase | • 6-amino penicillinanic acid and 7-ADCA | penicillin G or V | *Enz Eng* **6:** 291 (1982) *Enz Eng* **8:** 155 |
| • hydroxysteroid dehydrogenases | • steroid transformations | | Carrea *et al. Methods in Enzymology* **136**: 150 (1987) |
| • 5'phosphodiesterase, nucleases | • 5'-ribonucleotides | | Keller *et al. Methods in Enzymology* **136**: 517 (1987) |
| •esterase | • β-lactam precursor (chiral mono esters eg ßamino glutaric acid monoalkyl ester) | corresponding diesters | Japanese patent application: 82-159, 493 (1981) Biseibutsu Company |
| • lipases | • β-blockers | | Kloosterman,M *et al., Trends in Biotechnology* **6:** 251-256 (1988) |

Production of acrylamide using CLEC technology

The following is a description of one use of the method of the present invention: the adaptation of acrylamide production from immobilized cells which overproduce nitrile hydratase enzyme (Nagasawa, T. and Yamada, H., Trends in Biotechnology 7: 153-158 (1989)) to the CLEC technology previously disclosed herein.

Industrial scale production of acrylamide, an important commodity chemical, has been described by Yamada and collaborators (Nagasawa, T. and Yamada, H., Trends in Biotechnology 7: 153-158 (1989)). Kilotons of acrylamide per year are produced in chemical reactors loaded with entrapped cells selected as overproducers of the enzyme nitrile hydratase. Nitrile hydratase has also been reported as purified and crystallized from two sources, Brevibacterium R312 (Nagasawa et. al., Biochem. Biophys. Res. Commun. 139: 1305-1312 (1986) and P. chlororaphis B23 (Magasawa et. al., Eur. J. Biochem. 162: 691-698 (1987). As disclosed here, these crystalline enzymes can each be immobilized by crosslinking with glutaraldehyde or other suitable crosslinking reagent to produce a CLEC. The nitrile hydratase CLECs can then be used in a conventional reactor, in place of the entrapped cells currently used. Adapting this process to CLEC technology leads to immediate advantages. These include; reduced plant size and improved throughput resulting from the enhanced activity per unit volume implicit in the higher enzyme concentration in the CLECs, and improved substrate and product diffusion rates; reduction in undesired contamination and side reactions, resulting from the higher purity of CLECs; and reduced sensitivity to microbial contamination in the absence of cells. In addition, there are other benefits available only to a CLEC-based method. These benefits include: higher temperature operation to improve reaction rates; the ability to operate in aqueous, organic and near-anhydrous solvents, allowing optimization of the acrylamide production reaction; and enhanced half-life in operation and storage, resulting from the higher chemical and mechanical stability of CLECs, particularly in unconventional solvents.

Medical applications of CLEC technology - extracorporeal treatment

The method of the present invention and an appropriately selected CLEC or set of CLECs can also be used for medical applications. A CLEC or a set of CLECs can be used, for example, to remove a component of a fluid, such as blood, generally by altering the component, and thus, converting it to a substance not detrimental to an individual or which can be removed by normal body processes (e.g., via detoxification, or degradation in the liver, excretion via the kidneys). In this application, an appropriately-selected CLEC or set of CLECs is brought into contact with body fluid, which contains the component to be altered, or a reactant (product or substrate) of a reaction in which the component participates, upon which the enzyme in the CLEC acts. As a result, the enzyme is able to act upon the component to be altered or with another substance which is a product of a reaction in which the component to be altered participates. The activity of the enzyme results in direct alteration of the component to be removed or in alteration of the product of the reaction in which the component participates (thus making continuation of the reaction impossible). This can be carried out through the use of an extracorporeal device which includes an appropriately-selected CLEC or set of CLECs and a retaining means which is made of a material; such as a porous material on which a CLEC is retained or a tube in which a CLEC is present, which allows contact between the component itself or the substance in the fluid which is a product of a reaction in which the component to be altered participates.

This might also be achieved by the insertion of an appropriate CLEC into a suitable body compartment, such as the peritoneum or a lymph node, where the CLEC would have access to bodily fluids. This insertion might be done surgically, or by injection of the CLEC slurry. Direct injection of CLEC into the blood stream would not be appropriate, given the high risk of embolism.

The use of appropriate CLECs in this area might serve as an alternative to genetic methods in enzyme replacement therapy to correct a natural deficiency, such as, e.g. phenylketonuria.

Table 3 illustrates some of the medical applications in which CLECs could be used. For the majority of these cases, the extra-corporeal treatment is still in the research phase, but the benefits that CLECs offer could provide novel treatments in areas in which there was previously no alternative treatment.

TABLE 3

| Enzyme employed | Removal of:- | Disease/patients treated | References |
|---|---|---|---|
| • asparaginase | • asparagine | • leukemia (Removal of asparagine, an important cancer nutrient, harms leukemic cells which cannot manufacture the essential amino acid - asparagine; normal cells can manufacture asparagine and so are unaffected by this treatment.) | Klein,M., Langer,R., *Trends in Biotechnology* **4:** 179-185 (1986) and references therein Chang, T.M.S., *Methods in Enzymology* **137:** 444-457 (1987) & references therein |
| • heparinase | • heparin | • deheparinization for hemoperfusion patients eg kidney dialysis | Langer, R.,*et al., Science* **217:** 261-263 (1982) |
| • bilirubin oxidase | • bilinibin | • neo-natal jaundice | Lavin,A.,*et al., Science* **230:** 543-545 (1985) |
| • carboxypeptidase | • methotrexate | • chemotherapy patients | Pitt,A.M.,*et al., Appl. Biochem.Biotechnol.***8:** 55-68 (1983) |
| • tyrosinase | • aromatic amino acids | • liver failure exhibiting pathological elevations of amino acids | Chang.T.M.S., *Sem.Liver Dis.Ser.* **6:** 148 (1986) |
| • phenylalanine ammonium lyase | • phenylalanine | • phenylketonuria and liver failure | Ambrus,C.M., *et al., J. Pharm.& Exp.Ther.* **224:** 598-602 (1983) |
| • multi-enzyme system including: urease, glutamate dehydro-genase, glucose, dehydrogenase & a transaminase | • urea (converted into glutamic and other amino acids, via ammonia) | • detoxification for chronic renal failure patients | Chang,T.M.S., *Methods in Enzymology* **137:** 444-457 (1987) & references therein Chang,T.M.S., *Enzyme Eng* **5:** 225 (1980) |
| • arginase | • arginine | • familial hyperargininaemia | Kanalas.JJ. *et al., Biochem. Med.* **27:** 46-55 (1982) |
| • glutamate dehydrogenase & ammonia | • ammonia | • liver failure | Maugh,T.H., *Science* **223:** 474-476 (1984) |

A particular application of the present method is the heparin lyase system for blood deheparinization (Bernstein et al., Methods in Enzymology 137: 515-529 (1987)), which is discussed below.

All extracorporeal devices perfused with blood, such as kidney dialysis, continuous arteriovenous hemofiltration or extracorporeal membrane oxygenators, require heparinization of the patient to avoid blood clotting. However, heparinization of the patient leads to hemorrhagic complications and remains a threat to human safety. These problems increase as the perfusion times increase, for example with the membrane oxygenator, and can lead to serious bleeding. After extracorporeal therapy, heparin may be removed from blood by employing a heparinase device at the effluent of the extracorporeal device which eliminates all heparin from the blood returned to the patient and thus avoids the current problems of heparinization.

Published research (Langer et al. Science 217: 261-263 (1982)); Bernstein et al., Methods in Enzymology 137: 515-529 (1987)), details the problems presented by conventionally immobilized enzymes used in extracorporeal devices. The principal problem is that conventional immobilization results in a low retention of enzyme activity per unit volume, thus requiring a large volume of immobilized enzyme to perform the necessary heparinization. This volume is too large to be of practical use in humans. However, the high retention of activity per unit volume in CLECs, due to the lack of inert support circumvents this problem and offers a practical solution to deheparinization of humans. The enhanced stability of CLECs will reduce the disassociation of enzyme from the crosslinked crystal. This is superior to the less stable, conventionally immobilized enzymes, because immune responses resulting from enzyme leakage will be reduced. CLEC temperature stability prevents denaturation of the enzyme due to high transient temperatures during storage; it is likely that CLECs may retain high activity, even when stored at room temperature. In addition, CLECs will be cheaper and more convenient to use than their conventionally immobilized counterparts because of their longer operational and storage lifetimes.

Additional applications of CLEC technology: biosensors

A CLEC or a set of CLECs can be used as a component of a sensor, referred to as a biosensor, useful for detecting and/or quantitating an analyte of interest in a fluid, such as body fluid (e.g., blood, urine), chemical and laboratory reaction media, organic media, water, culture medium and beverages. In some instances, the fluid in question can be a gas, as in an alcohol breath analyzer (Barzana, E., Klibanov, A., and Karell, M., NASA Tech Briefs 13:104 (1989)). In this application an appropriately-selected CLEC or set of CLECs is brought into contact with a fluid to be analyzed for the analyte of interest. The analyte of interest can be measured directly (e.g., blood glucose level) or indirectly (e.g., by detecting or quantitating a substance which is a reactant (product or substrate) in a reaction in which the analyte of interest participates). In either case, the CLEC is able to act upon the analyte or the substance which is a reactant in a reaction in which the analyte also participates. The activity of the enzyme results in a detectable change (e.g., change in pH, production of light, heat, change in electrical potential) which is detected and/or quantitated by an appropriate detecting means (e.g., pH electrode, light or heat sensing device, means for measuring electrical charge) (Janata, J., et al., Anal. Chem. 62: 33R-44R (1990)). Any means useful for detecting the change resulting from the enzyme-catalyzed method can be used. A biosensor of the present invention includes a CLEC or set of CLECs and a retaining means for the CLEC which allows contact between the CLEC(s) and the analyte of interest or the substance in the fluid which is a reactant in the reaction in which the analyte of interest participates.

Table 4 illustrates some of the biosensor applications in which CLECs could be used. Currently immobilized enzymes are used in these applications, but suffer from low stability, low enzyme density, short lifetimes and lack of reproducibility. These examples can be readily adapted to the CLEC technology disclosed here.

Table 4

| Enzyme Employed | Detection of:- | Application | Reference |
|---|---|---|---|
| • glucose oxidase | • glucose | • diabetics | • Daniles,B.,Mossbach,K, *Methods in Enzymology* **137:** 4-7 (1987)<br>• Hall,E *"Biosensors"* Open University Press (1990)<br>• Taylor,R., *Proceed. Biotechnology Conference* 1989; 275-287<br>• Anthony *et al.,* "*Biosensors, Fundamentals and Applications*", Oxford University Press (1987) |
| • creatinine deiminase | • creatinine | • kidney function | • Tabata,M. *et al., Anal. Biochem.* **134:** 44 (1983) |
| • urease | • urea | • kidney function | • Hsuie,G.H *et al.*, *Polym. Mater.Sci.Eng.* ***57:*** 825-829 (1987)<br>• Kobos, *et al. Anal.Chem.* **60**: 1996-1998 (1988) |

Table 4 (continued)

| **Enzyme Employed** | **Detection of:-** | **Application** | **Reference** |
|---|---|---|---|
| • lactate oxidase & dehydrogenase | • lactate | • clinical applications | • Blaedel, W.J. & Jenkins, R. A., *Anal. Chem.* **48(8):** 1240 (1976)<br>• Sagaguchi, Y.,*et al., J. Appl. Biochem* **3:** 32 (1981) |
| • glucose-6-pyruvate dehydrogenase | • Glucose-6-phosphate, sucrose and ATP | • diabetics and other medical | • ibid as glucose oxidase |
| • alcohol dehydrogenase, alcohol oxidase | • ethanol & other alcohols: acetic, formic acids | • breathalysers and industrial applications | • Romette,J.L.*et al., Methods in Enzymology* **137:** 217-225 (1987)<br>• Ho,M.H., *Methods in Enzymology* ***137:*** 271-288 (1987) |
| • β-fructosidase | • sucrose | • industrial applications | • Romette, J.L. *et al., Methods in Enzymology* **137**: 217-225 (1987) |
| • cholesterol oxidase | • cholesterol | • cholesterol testing | • Satoh,I.*Methods in Enzymology* **137**: 217-225 (1987) |
| • catalase | • uric acid, cholesterol | • atherosclerotic and other medical | • Satoh,I.*Methods in Enzymology* **137:** 217-225 (1987) |
| • carboxy peptidase | • methotrexate | • cancer | • ibid as glucose oxidase |
| • carbonic anhydrase | • carbon dioxide | • industrial, laboratory & environmental applications | • ibid as glucose oxidase |
| • L-amino acid oxidase | • amino acids | • medical and industrial | • ibid as glucose oxidase |
| • β-lactamase penicillinase | • penicillin | • medical | • Anzai *et al., Bull.Chem. Soc.Jpn*. **60:** 4133-4137 (1988) |
| • alkaline phosphatase | • phosphate | • metabolite monitoring | • ibid as glucose oxidase |
| • nitrate/nitrite reductase | • nitrates & nitrites | • metabolite and food monitoring | • ibid as glucose oxidase |
| • arylsulfatase | • sulfate | • metabolite monitoring | • ibid as glucose oxidase |
| • succinate dehydrogenase | • succinate | • industrial | • ibid as glucose oxidase |
| • bacterial luciferase | $FMNH_2$ and coupled reactions | • detection of $10^{-18}$ molar quantities of $FMNH_2$ through measurement of photon measurement of photon | • WannlundJ., *et al.*, "Luminescent assays: Perspectives in endocrinology and clinical chemistry"; Eds Serio,M. and Pazzagli,M. 1:125 (1982)<br>• Kurkijarvi *et al.*, *Methods in Enzymology* **137**: 171-181 (1987) |

Table 4 (continued)

| Enzyme Employed | Detection of:- | Application | Reference |
|---|---|---|---|
| • firefly luciferase | ATP and coupled reactions | • detection of $10^{-12}$ molar quantities of ATP through measurement of photon release | • Kurkijarvi *et al.*, *Methods in Enzymology* **137:** 171-181(1987) · Murachi *et al.*, *Methods in Enzymology* **137:** 260-271 (1988) |

In the method of the present invention as it is carried out for the analysis of samples in a biosensor, it is particularly desirable to produce the largest possible detectable signal from the smallest possible quantity of substrate and catalyst. In this regard, the CLEC technology disclosed here is particularly attractive, since it achieves the highest possible concentration of enzyme catalyst in a given volume.

Often, considerable efforts are made to couple an ultimate enzymatic reaction of interest, either directly, or through suitable intermediates, to the production of light by enzymes like luciferase (Kurkijarvi et al., Methods in Enzymol. 137: 171-181 (1988)). This is done in order to take advantage of the unparalleled sensitivity and efficiency of photon detection equipment, which allows for the detection of femtomolar concentrations of enzyme reaction products under appropriate conditions. Following this principle, biosensor systems have been designed, using conventionally immobilized enzymes, to detect various substrates of clinical and other interest. Light producing reactions have been coupled to assay reactions detecting substrates like, D-glucose, L-lactate, L-glutamate and ethanol, among others, at extremely low concentration.

With regard to this application, the luciferase enzyme from Vibrio harveyii has been reported as crystallized (Swanson et al., J. Biol. Chem. 260: 1287-1289 (1985)). Crystals of this luciferase can be crosslinked with glutaraldehyde or other suitable reagent to form a CLEC of luciferase. For biosensor and analytical uses, a CLEC of luciferase offers many advantages over conventionally immobilized enzyme. In a CLEC, the entire volume of the luciferase CLEC would consist of light emitting enzyme. In a conventionally immobilized enzyme system, however, as much as 95% of the total volume is taken up by "inert" carrier material, which more likely functions as an absorber of the light emitted by enzyme. In addition, the enhanced stability of CLECs should facilitate storage at room temperature, and also makes possible novel sensing applications in harsh environments and elevated temperatures.

Additional applications of CLEC technology - laboratory reactions

CLECs may be used as laboratory reagents in small columns or in batch processes, which can be used to carry out laboratory reactions. Some of the broad categories of reactions are in Table 5.

Table 5

| Enzyme Employed | Reaction type catalyzed | Reference |
|---|---|---|
| • lipases, phospholipases | • Stereoselective synthesis: including esterification, transesterification, aminolysis, lactonizations, polycondensations, acylation, oximolysis and resolution of racemic mixtures | • Zaks.A.& Klibanov,A.M. *Proc.Nat. Acad.Sci.USA.* **82**: 3192-3196 (1985)<br>• Klibanov,A.M. *Acc.Chem.Res.* **23:** 114-120 (1990) and references therein · Wong,C.H.,*Chemtracts-Organic Chemistry* **3**:91-111 (1990) and references therein |
| • esterases | • stereoselective synthesis and resolution | • Kobayashi et al., *Tetrahedron Letters* **Vol 25**, **#24**: 2557-2560 (1984)<br>• Schneider et al., *Agnew. Chem. Int. Ed.Ensl.***23** (#1): 64-68 (1984) |
| • tyrosinase | • oxidation of phenols to produce quinones | • Kazandjian,R.Z and Klibanov, A.M. *J.Am.Chem.Soc* **110:** 584-589 (1986) |

Table 5 (continued)

| Enzyme Employed | Reaction type catalyzed | Reference |
|---|---|---|
| • proteases, eg subtilisin | • stereoselective acylation of carbohydrates | • Riva et al. *J.Am.Chem.Soc.***110**: 584-589 (1988) |
| • oxidases | • selective oxidation of hydrocarbons | • Klibanov,A.M. *Acc.Chem.Res.* **23:** 114-120 (1990) and references therein |
| • other enzymes not requiring co-factors: isomerases, lyases, aldolases, glycosyl transferases, glycosidases | • Stereoselective synthesis: | • Wong,C.H.,*Chemtracts-Organic Chemistry* **3**:91-111 (1990) and references therein |
| • other enzymes not requiring added co-factors: flavoenzymes, pyridoxal phosphate enzymes, metalloenzymes | • Stereoselective synthesis: | • Wong,C.H.,*Chemtracts-Organic Chemistry* **3**:91-111 (1990) and references therein |
| • enzymes requiring co-factors: kinases (ATP), oxidoreductatses (NAD/P), methyl transferases (SAM), CoA-requiring enzymes, sulfurases (PAPS) | • Stereoselective synthesis: | • Wong,C.H.,*Chemtracts-Organic Chemistry* **3**:91-111 (1990) and references therein |

Schneider et al. (Agnew. Chem. Int. Ed. Engl. 23 (No.1): 64-68 (1984)) illustrates how enzymes may be used in organic syntheses. Pig liver esterase was employed in the meso-ester transformation into a chiral mono-ester in an aqueous phosphate buffer.

The advantages of CLEC catalyzed reactions for laboratory use are threefold. First, CLEC retain high activity in harsh environments (eg. aqueous, organic, near-anhydrous solvents and mixtures of these, and at high temperatures) that are typical of laboratory chemical synthesis experiments. Second, CLEC exhibit high operational and storage stability, which is appropriate for intermittent laboratory experiments. Third, their high activity per unit volume will allow shorter reaction times and require smaller volumes of enzyme (per unit of activity). Thus, the advantages that CLECs offer over free or immobilized enzymes, provide organic chemists with an alternative, highly selective, synthetic tool.

In all of these instances described above, but not limited to these, the method of this invention can be adapted by one of ordinary skill in the art, to convert a process using a conventionally immobilized enzyme catalyst to the use of a CLEC of the appropriate enzyme. CLECs can not only replace conventional immobilized enzymes, but also be used in cell mediated transformations. The present invention will now be illustrated by the following Examples which are not intended to be limiting in any way.

EXAMPLE 1

Crystallization and crosslinking of thermolysin for synthesis of the aspartame precursor, Z-Asp-Phe-OMe.

Crystallization

250 mg of thermolysin from Bacillus thermoproteolyticus was purchased from Boehringer-Mannheim GmbH, and dissolved in 4 ml of 45% dimethyl sulfoxide (DMSO) and 55% 1.40 M calcium acetate, 0.50 M sodium cacodylate at pH 6.5. These starting conditions are similar to those described by Matthews et. al. for the production of diffraction quality thermolysin crystals (see, eg., Holmes and Matthews, J. Mol. Biol. 160: 623-639 (1982)). The protein solution was then concentrated to 1 ml in a Centricon 10 micro-concentrator. A good yield of microcrystals was obtained by a process of flash crystallization, now disclosed here, in which 1 ml of water, or 1.40 M calcium acetate, 0.50 M sodium cacodylate at pH 6.5, was rapidly injected into either of the thermolysin-DMSO solutions described above. A shower of hexagonal micro-crystals of approximately uniform dimensions (approx. $10^{-1}$ mm in length) results from this process.

**Crosslinking of thermolysin microcrystals.**

The protocol used in this specific example of the method of this invention is an adaptation of that described by Nakanishi et. al. (Biotechnology 3: 459-464 (1985)), in which protocol, thermolysin was first adsorbed onto a carrier bead composed of the ion-exchange resin Amberlite XAD-7, and subsequently immobilized by cross-linking with glutaraldehyde (Quiocho and Richards, Proc. Natl. Acad. Sci. (USA) 52:833-839 (1964)). In this exemplification, the microcrystals of thermolysin obtained above were centrifuged and pelleted, and the supernatant was discarded. 5 ml of 17.5% technical grade glutaraldehyde, in 2.5 % DMSO, 0.05M calcium acetate, and 0.025M sodium cacodylate at pH 6.5, were then added to the microcrystals. The mixture was incubated with gentle agitation at 37°C for 4 hours. The crosslinking reaction was stopped by repeated washing of the crystals with 10 ml aliquots of water to remove the glutaraldehyde solution. The washed cross-linked thermolysin crystals constitute the thermolysin CLEC used below as a catalyst.

Synthesis of Z-Asp-Phe-OMe in an aqueous solution

5 ml of a thermolysin CLEC suspension were added to a continuous stirred-batch reactor incubated at 37°C. After centrifugation and decantation of the supernatant, an aqueous reaction mixture was added to the CLECs. This solution was prepared by mixing 80 mg of Z-L-Asp and 80 mg of L-Phe-OMe-HCl in 1 ml of water, with acetic acid added to obtain a pH of 7.0. Samples were taken for analysis by HPLC. Table 6 below shows the HPLC peak height of the Z-L-Asp substrate peak after the indicated time of reaction, normalized to 1 at time t=0. Since Z-L-Asp is rate limiting in this reaction, measuring its depletion is equivalent to measuring the appearance of product Z-L-Asp-L-Phe-OMe (Nakanishi et. al. Biotechnology 3: 459-464 (1985)). Table 6 also includes the normalized peak height of limiting Z-L-Asp substrate remaining, and an estimate of the degree of completion of the reaction. It is clear that the reaction proceeded to about 20% completion within the first 30 seconds and plateaued there. These results are consistent with the observations of Nakanishi et al. (Biotechnology 3: 459-464 (1985)) when using conventionally immobilized thermolysin in an aqueous reaction mixture as above, and are attributable to the sparing solubility of the Z-L-Asp-L-Phe-OMe product in water.

TABLE 6

| Reaction Time (sec) | Peak Height (Normalized) | Percent Completion |
|---|---|---|
| 0 | 1.000 | |
| 30 | 0.727 | 27.3% |
| 60 | 0.857 | 14.3% |
| 120 | 0.940 | 6.0% |
| 180 | 0.797 | 20.3% |

Synthesis of Z-Asp-Phe-OMe in a near-anhydrous solution

5 ml of a thermolysin CLEC suspension were added to a continuous stirred-batch reactor incubated at 37°C. After centrifugation and decantation of the supernatant, a near-anhydrous organic reaction mixture was added to the CLECs. This solution was prepared by mixing 80 mg of Z-L-Asp and 240 mg of L-Phe-OMe in 1 ml of 99% ethyl acetate and 1% water. Samples were taken for analysis by HPLC. Table 7 below shows the HPLC peak height of the Z-L-Asp substrate peak after the indicated time of reaction, normalized to 1 at time t=0. Since Z-L-Asp is rate limiting in this reaction, measuring its depletion is equivalent to measuring the appearance of product Z-L-Asp-L-Phe-OMe (Nakanishi et. al. Biotechnology 3: 459-464 (1985)). Table #7 also includes the normalized peak height of limiting Z-L-Asp substrate remaining, and an estimate of the degree of completion of the reaction. In this case, the reaction proceeded to about 70% completion within the first 30 seconds and plateaued there. These results are also consistent with the observations of Nakanishi et al. (Biotechnology 3: 459-464 (1985)) with conventionally immobilized thermolysin in a near-anhydrous reaction mixture, and are attributable to product inhibition of the enzyme.

TABLE 7

| Reaction Time (sec) | Peak Height (Normalized) | Percent Completion |
|---|---|---|
| 0 | 1.000 | |
| 30 | 0.323 | 67.7% |
| 60 | 0.314 | 68.6% |
| 120 | 0.305 | 69.5% |
| 180 | 0.272 | 72.8% |

**EXAMPLE 2**

**Crystallization, cross-linking and lyophilization of thermolysin and assessment of characteristics of resulting product**

Crystallization of thermolysin

Thermolysin (Diawa Kasei K.K., Japan) was dissolved in 10 mM calcium acetate (Sigma), pH 10.0, to a concentration of 10% (w/v). The pH of the solution was maintained at 10.0 by titration with 2 M NaOH. Following complete solubilization, the protein solution was titrated to pH 8.0 with 2 M HCl. Solid calcium acetate was added to 1.2 M. Dimethyl sulfoxide (Sigma) was then added to 30%. The protein was concentrated to 100 mg/ml by ultrafiltration in an Amicon stir cell (10,000 MWCO membrane). Concentrated enzyme was aliquoted and stored at -70°C. Thermolysin was crystallized by the addition of 9 volumes demineralized water to 1 volume concentrated (100 mg/ml) protein solution. The solution was briefly vortexed and allowed to stand overnight at room temperature. Crystals were washed with 10 volumes of 10 Mm calcium acetate pH 7.0 and recovered by low speed centrifugation (10 min at 1500 x G, Beckman GPR centrifuge).

The rapid addition of water to a concentrated (100 mg/ml) solution of thermolysin induces the formation of crystals which become visible under low-power magnification within ten minutes. Crystal size is reproducibly dependent on the final protein concentration. Three volumes of water to one volume of thermolysin concentrate (100 mg/ml) will produce 0.5mm long, X-ray diffraction quality hexagonal rods that correspond to the crystals described earlier by Colman et al. (Colman, P.M., Jansonius, J.N. and Matthews, B.W., J. Mol. Biol. 70: 701-724 (1972)), as confirmed by us by diffraction analysis. Adding ten volumes of water to one of protein concentrate reduces the length of the resulting crystals to 0.05mm. These micro-crystals are preferred in CLEC applications, since they tend to minimize diffusion problems related to crystal size (see eg., Quiocho, F.A. and Richards, F.M. Biochemistry 5: 4062-4076 (1967)). Within a given batch of protein, crystal size was consistently uniform. (Crystals 0.05 - 0.10 mm in length were used in this study to facilitate accurate pipetting of crystalline suspensions.) Densitometer scans of SDS-PAGE showed a six-fold purification of the enzyme on crystallization, significantly increasing the specific activity of the CLECs. Crystallization resulted in a 20% decrease in the total activity of the CLEC protein compared to soluble thermolysin, when assayed by spectrophotometric cleavage of the dipeptide substrate furylacryloyl-glycyl-L-leucine-amide (FAGLA), as described below.

Crosslinking of thermolysin crystals

Thermolysin crystals were crosslinked for 3 hours at room temperature in a solution of 12.5% glutaraldehyde (Sigma), 5% DMSO and 50 mM Tris pH 6.5. The crosslinked crystals were washed 3 times in demineralized water and recovered by low speed centrifugation, as described in respect to crystallization of thermolysin. Chemical cross-linking of enzyme crystals stabilizes the crystal lattice and the constituent enzyme molecules in the crystal sufficiently so as to permit the practical use of CLECs in environments that are otherwise incompatible with enzyme function. There was no measurable difference in enzymatic activity between the crosslinked and uncrosslinked crystals when assayed (spectrophotometrically) by monitoring cleavage of the dipeptide substrate FAGLA (described below). Moreover, crosslinking stabilizes CLECs to the point that they can be lyophilized, with retention of full enzymatic activity upon reconstitution in aqueous, organic, and mixed aqueous-organic solvents as shown in Figure 1 and Table 8. Although crystallization resulted in a 30% decrease in the specific activity of the CLEC protein compared to soluble thermolysin, crosslinking and lyophilization of the CLECs did not further diminish specific activity.

TABLE 8 -

| Thermolysin Activity | | | |
|---|---|---|---|
| | | Absorbance 345 nm | |
| | Time (min) | CLEC | Soluble Enzyme |
| 1 | 0.0 | 0.314 | 0.315 |
| 2 | 1.0 | 0.272 | 0.271 |
| 3 | 3.0 | 0.235 | 0.218 |
| 4 | 5.0 | 0.204 | 0.198 |
| 5 | 10.0 | 0.184 | 0.185 |
| 6 | 15.0 | 0.183 | 0.184 |

Enzymatic activity of soluble and CLEC thermolysin

The catalytic activity of soluble and CLEC thermolysin was assayed (Feder, J. and Schuck, J.M., Biochemistry 9: 2784-2791 (1970)) by hydrolysis of the blocked dipeptide substrate furylacryloyl-glycyl-L-leucine-amide (FAGLA) (Schweizerhall). Cleavage of the amide bond was measured spectrophotometrically by a decrease in absorbance at 345 nm. Initial enzyme concentration was $10^{-7}$M by Bradford protein determination and densitometer scanning (Pharmacia LKB UltroScan XL) of Coomassie stained SDS-PAGE gels. CLEC enzyme is defined as reconstituted lyophilized cross-linked thermolysin crystals. Soluble enzyme is defined as thermolysin concentrated to 100 mg/ml. Enzyme was added to a 5 ml reaction volume containing substrate. Aliquots of the reaction mix were removed at the indicated times, and absorbance at 345nm was measured. CLEC thermolysin was separated from the reaction mix by brief centrifugation (Beckman, microcentrifuge E) before reading absorbance. Absorbance was fitted to a pseudo first order rate equation and kcat/Km was calculated by dividing the fitted value by enzyme concentration (Multifit 2.0 Curve Fitting for the Apple Macintosh Computer, Day Computing P.O. Box 327, Milton, Cambridge CB4 6WL, U.K. (1990)).

pH Dependence and Stability

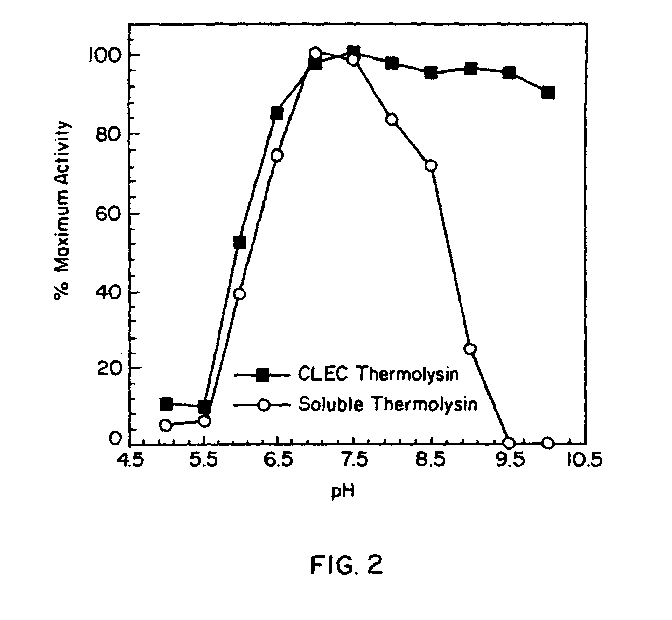

The pH optimum and stability of the soluble enzyme were compared to that of thermolysin CLECs by cleavage of the dipeptide substrate FAGLA. Results are shown in Figure 2 and Table 9. Both soluble and crystalline enzyme forms demonstrate maximum activity at pH 7. CLECs and soluble thermolysin also demonstrated similar activity in the acidic range and the bell shaped pH profile generated by the soluble enzyme was in good agreement with published data (Feder, J. and Schuck, J.M., Biochemistry 9: 2784-2791 (1970)). In the alkaline pH range, however, the crystalline enzyme maintains maximum activity, to pH 10, while the soluble enzyme has 75% activity at pH 8.5, and only 25% activity at pH 9. At pH 9.5, the soluble enzyme is completely inactive.

TABLE 9 -

| Thermolysin pH Curve | | | |
|---|---|---|---|
| | | % Maximum Activity | |
| | pH | CLEC | Soluble Enzyme |
| 1 | 5.0 | 10.250 | 5.170 |
| 2 | 5.5 | 9.750 | 6.070 |
| 3 | 6.0 | 52.500 | 39.100 |
| 4 | 6.5 | 85.000 | 74.610 |
| 5 | 7.0 | 97.500 | 100.000 |
| 6 | 7.5 | 100.000 | 98.650 |
| 7 | 8.0 | 97.500 | 82.920 |
| 8 | 8.5 | 95.000 | 71.910 |
| 9 | 9.0 | 96.250 | 24.720 |
| 10 | 9.5 | 95.000 | 0.000 |
| 11 | 10.0 | 90.000 | 0.000 |

Stability at elevated temperature

One can achieve higher reaction rates and lower diffusion times for substrates and products by operating a given chemical process at higher temperature, where one is usually limited by the temperature stability of substrates and products. In enzyme-based catalysis, however, it is often the loss of enzymatic activity that sets the practical limit on the temperature that a process can be run. The additional stabilization achieved in CLECs allows for enzymatic activity at much higher temperatures than is possible for soluble enzyme.

The enhanced stability at lower temperatures simplifies the routine long term storage of the CLEC catalysts. For example, it was necessary to store concentrated (>50mg/ml) solutions of soluble thermolysin at -80°C to retain maximum specific activity. At room temperature, activity was usually lost within one day. In contrast, rehydrated thermolysin CLECs could be routinely stored for months at room temperature with no apparent loss of activity. Unreconstituted lyophilized CLECs of thermolysin appear to be viable indefinitely.

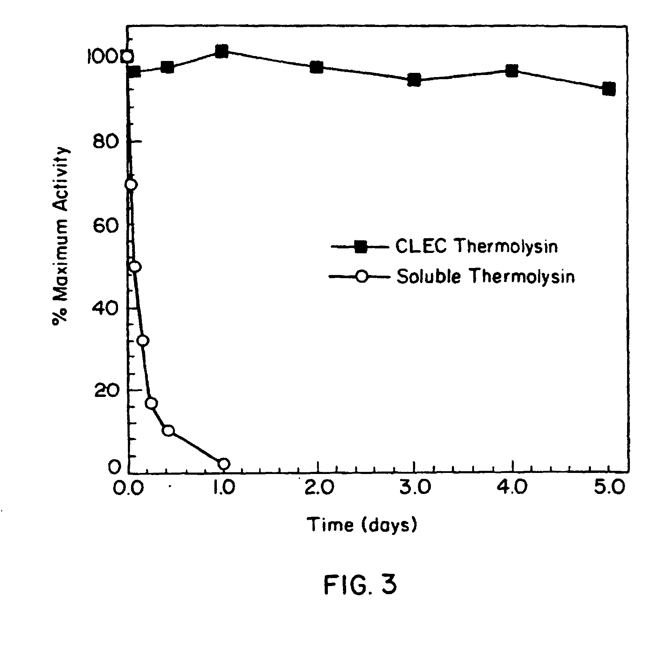

Thermal stability and resistance to autolysis were demonstrated in thermolysin CLECs following incubation at 65°C for five consecutive days (Figure 3 and Table 10). Thermolysin CLECs retained maximum activity after five days incubation at elevated temperature. In contrast, the soluble thermolysin lost 50% of its initial activity after only two hours incubation and demonstrated negligible activity after 24 hours incubation at 65°C.

TABLE 10 -

| Thermolysin Thermal Stability at 65°C % Maximum Activity | | | |
|---|---|---|---|
| | Time (days) | CLEC | Soluble Enzyme |
| 1 | 0.000 | 100.000 | 100.000 |
| 2 | 0.041 | | 70.000 |
| 3 | 0.083 | 96.000 | 50.000 |
| 4 | 0.164 | | 32.000 |
| 5 | 0.246 | | 17.000 |
| 6 | 0.410 | 97.0 | 10.000 |
| 7 | 1.000 | 101.0 | 2.000 |
| 8 | 2.000 | 97.0 | |
| 9 | 3.000 | 94.0 | |
| 10 | 4.000 | 96.0 | |
| 11 | 5.000 | 92.0 | |

The activity of soluble and CLEC thermolysin was measured following incubation at 65°C. Soluble thermolysin was incubated in 10 mM calcium acetate, 50 mM Tris pH 7.0 in a 65°C water bath. The reaction volume was 500µl. Final protein concentration was 10 mg/ml. Aliquots were removed at times 0, 1, 2, 4, 6, 10, and 18 hours. The samples were assayed by SDS-PAGE and FAGLA cleavage at room temperature as described above. For the thermolysin CLECs, a 250 µl crystal suspension in 10mM calcium acetate and 50 mM Tris was also incubated in a 65°C water bath. Activity was assayed at times 0, 1, 6, 24, 48, 72, 96, and 120 hours by FAGLA cleavage.

Resistance to exogenous proteolysis

Assessment of the resistance of the thermolysin CLEC to the action of an exogenous protease was also carried out. SDS-PAGE (Sodium dodecyl sulfate poly acrylamide gel electrophoresis) analysis suggests that commercial enzymes can contain a substantial percentage of contaminants, some of which might have proteolytic activity against the principal soluble enzyme species. Given the packing of enzyme molecules in a crystal lattice one might assume that the interior enzyme molecules in a CLEC would be protected from proteolysis. To test this possibility, thermolysin CLECs and a soluble enzyme preparation were incubated in the presence of the streptococcal protease, Pronase®, a nonspecific protease capable of digesting most proteins to free amino acids (Calbiochem 1990 Catalog; LaJolla, CA).

Soluble and CLEC thermolysin were incubated in 50mM Tris, pH 7.5, at 40°C in the presence of the protease Pronase® (Calbiochem). The Pronase® to thermolysin ratio was 1/40. To inhibit thermolysin autolysis and prevent the proteolytic destruction of pronase by the thermolysin, EDTA was added to the soluble enzyme reaction to a final concentration of 100 mM (EDTA inhibits thermolysin activity but not Pronase®). At the times indicated aliquots were removed from the reaction mix and activity was assayed spectrophotometrically by cleavage of the dipeptide substrates FAGLA. To offset thermolysin inhibition due to the presence of EDTA, the spectrophotometric assay of soluble enzyme activity was performed in 0.5 M calcium acetate buffer pH 7.0 and enzyme concentration was increased two fold. Crosslinked crystalline enzyme was assayed as described above.

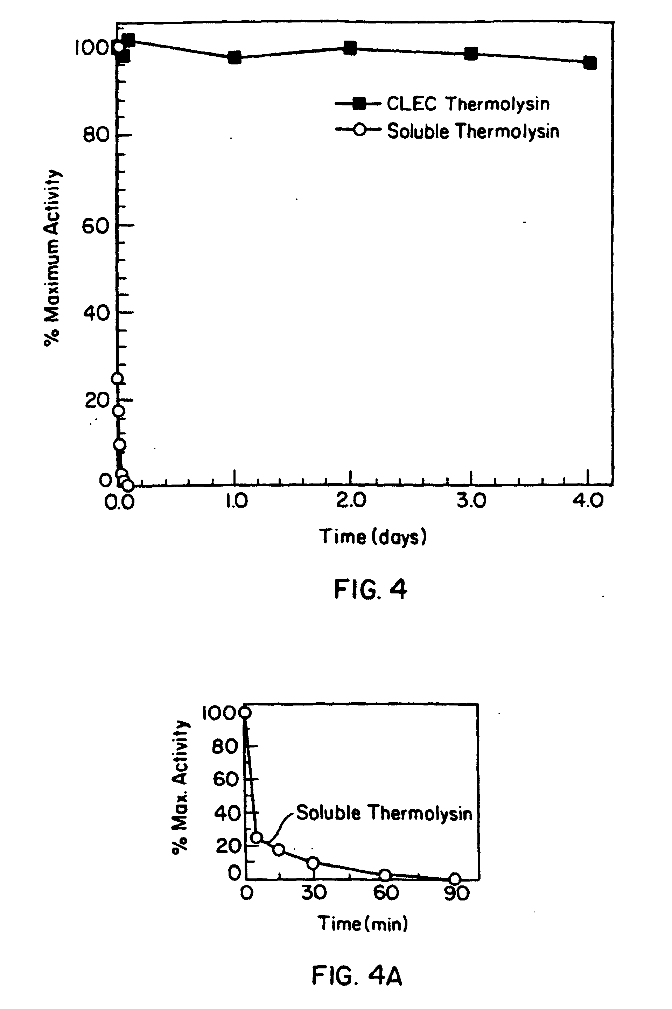

As can be seen in Figure 4 and Table 11, the soluble thermolysin was rapidly degraded and lost all activity after 90 minutes incubation. In contrast, the activity of the thermolysin CLEC was unaffected by four days incubation in the presence of protease. This near imperviousness to proteolysis is of particular interest in diagnostic biosensor applications where a suitable CLEC might be called upon to act in the presence of an unknown cocktail of naturally occurring proteolytic enzymes.

TABLE 11

| Protease Resistance | | | | |
|---|---|---|---|---|
| | Time (days) | % Maximum Activity | | Time (min) |
| | | CLEC | Soluble Enzyme | |
| 1 | 0.000 | 100.0 | 100.0 | 0.000 |
| 2 | 0.003 | | 25.0 | 5.000 |
| 3 | 0.010 | | 17.5 | 15.000 |
| 4 | 0.021 | | 9.5 | 30.000 |
| 5 | 0.042 | 98.0 | 3.0 | 60.000 |
| 6 | 0.063 | | 1.0 | 90.000 |
| 7 | 0.084 | 101.0 | 0.0 | |
| 8 | 1.000 | 97.0 | | |
| 9 | 2.000 | 99.0 | | |
| 10 | 3.000 | 98.0 | | |
| 11 | 4.000 | 96.0 | | |

Stability in the presence of organic solvent

In order for enzymes to gain ideal acceptance as viable industrial catalysts, they must be able to function without excessive intervention in the practical environment of manufacturing processes. In particular, this would include the use of aqueous, polar and non-polar organic solvents, and mixtures of these. In commercial applications, aqueous-organic solvent mixtures allow manipulation of product formation by taking advantage of relative solubilities of products and substrates.

Soluble thermolysin and thermolysin CLECs exhibited markedly different stability in the presence of organic solvents. (Table 12). Soluble enzyme concentrations which could be incubated in organic solvent were limited to a maximum of 10 mg/ml. Concentrations greater than this value resulted in the instantaneous precipitation of thermolysin upon addition of organic solvent. In contrast, thermolysin CLEC concentrations were limited only by the volume occupied by the crystals. Soluble thermolysin retained the greatest activity (75%) following incubation in acetone, and the least (36%) in tetrahydrofuran. Following a one hour incubation in the presence of acetonitrile or dioxane the soluble enzyme lost approximately 50% of its initial activity. The CLEC thermolysin retained greater than 95% maximum activity following incubation with all organics assayed.

TABLE 12

| | % Maximum Activity | |
|---|---|---|
| | Soluble Enzyme | CLEC |
| **Acetonitrile** | **42** | **102** |
| **Dioxane** | **66** | **97** |
| **Acetone** | **75** | **99** |
| **THF*** | **36** | **96** |

*** Tetrahydro Furan**

Stability in organic solvents

Thermolysin CLECs or soluble thermolysin preparations were incubated in 50% (v/v) solutions of the indicated organic solvents. A 100 µl slurry of thermolysin CLECs (10 mg/ml) in 10mM Tris pH 7 was placed in a 1/2 dram glass vial. An equal volume of the indicated organic solvent was added and the mixture was briefly vortexed. Twenty µl of soluble thermolysin (100 mg/ml) was diluted in 80 µl of 0.015M Tris buffer pH 7.0 in a 1/2 dram glass vial. A 100 µl volume of organic solvent was then added to the protein solution and briefly vortexed. CLEC and soluble enzyme were incubated in the presence of organic solvent for one hour at 40°C. Following incubation, enzyme activity was assayed by cleavage of the dipeptide substrate FAGLA as described.

Low water concentration is thought to disfavor unfolding to intermediate states on the path to enzyme denaturation. In CLECs, this restriction of conformational mobility is provided by the intermolecular contacts and cross-links between the constituent enzyme molecules making up the crystal lattice, rather than by the near-absence of water in the medium. As a result, intermediate water-organic solvent concentrations are readily tolerated by enzymes when formulated as CLECs, something previously unobserved with enzymes (see Table 12). This discovery opens up whole new areas of synthetic chemistry to exploitation using enzyme catalysis.

Even in near-anhydrous organic solvents, however, the routine use of enzymes has been hampered by their tendency to form ill-defined suspensions that are subject to clumping and other aggregation problems. This property makes these preparations inherently unattractive for large scale industrial processes. In contrast, CLECs and the constituent enzymes within the crystal lattice, remain mono-disperse in all these solvents.

Comparison with other immobilization methods

A number of useful reviews of enzyme immobilization methods have appeared in the literature (Maugh, T.H., Science, 223: 474-476 (1984)); Tramper, J., Trends in Biotechnology 3: 45-50 (1985)) . In these, the enzyme always represents a small fraction of the total volume of the immobilized particle, the bulk of it being inert carrier material. The carrier increases the mean free path between the solvent exterior of the immobilized enzyme particle and the enzyme active sites, exacerbating diffusion problems (Quiocho, F.A. and Richards, F.M., Biochemistry 5: 4062-4076 (1967)).

In a CLEC, the cross-linked crystal matrix provides its own support, eliminating the need for a carrier. As a result, the concentration of enzyme in a CLEC is close to the theoretical packing limit that can be achieved for molecules of a given size, greatly exceeding densities achievable even in concentrated solutions. The entire CLEC consists of active enzyme, and thus, the diffusion-related reduction of enzyme reaction rates usually observed with conventionally immobilized enzymes relative to enzymes in solution are minimized (See Figure 1), since the mean free path for substrate and product between active enzyme and free solvent will be greatly shortened for CLECs (compared to a conventional immobilized enzyme carrier particles). Importantly, the constituent enzyme in CLECs is intrinsically mono-disperse, and can be recovered by simple manipulations of the CLEC particles, such as filtration, centrifugation or decantation of solvent.

EXAMPLE 3

Crystallization, cross-linking and lyophilization of elastase and assessment of characteristics of the resulting product.

Crystallization of elastase

Lyophilized porcine pancreatic elastase (Serva) was dissolved in 0.1 M sodium acetate pH 5.0 to a concentration of 5 mg/ml (w/v) at room temperature. Rod shaped elastase crystals were visible within one minute of the complete solvation of the protein. The crystallization solution was transferred to 4°C and crystallization was completed overnight. Crystals were recovered by centrifugation as previously described.

Cross-linking of elastase crystals

A 200 µl volume of elastase crystals was added to a 1.3 ml solution of 5.77% glutaraldehyde and 1.5 M sodium acetate pH 5.0. The crystals were crosslinked for one hour with mild agitation (stir plate). Following cross-linking the crystals were washed with three 15 ml volumes of 0.2 M Tris pH 8.0. The elastase CLEC was lyophilized as described in Example 2.

Enzymatic activity of soluble and CLEC elastase

The catalytic activity of soluble and CLEC elastase was assayed spectrophotometrically by measuring hydrolysis of the substrate succinyl - $(Ala)_3$ p-nitroanilide (Bachem) [Bieth, et al. Biochem. Med 11:350-357 (1974)] (Table 13 Figure 5). Cleavage was monitored by increasing absorbance at 410 nm. Initial substrate concentration was 2 X $10^{-4}$. Enzyme concentration was 2.8 X $10^{-7}$M. CLEC or soluble enzyme was added to a 5 ml reaction volume containing substrate in 0.2 M Tris pH 8.0. As described previously, CLEC enzyme was removed from the reaction mix prior to measuring absorbance.

TABLE 13 -

| Elastase Activity | | | |
|---|---|---|---|
| | | Absorbance 400 nm | |
| | Time (min) | CLEC | Soluble Enzyme |
| 1 | 0.0 | 0.000 | 0.000 |
| 2 | 0.5 | 0.103 | 0.205 |
| 3 | 1.0 | 0.195 | 0.390 |
| 4 | 2.0 | 0.366 | 0.672 |
| 5 | 3.0 | 0.523 | 0.923 |
| 6 | 4.0 | 0.657 | 1.098 |
| 7 | 5.0 | 0.780 | 1.227 |
| 8 | 6.0 | 0.888 | 1.326 |
| 9 | 7.0 | 0.974 | 1.393 |
| 10 | 10.0 | 1.170 | 1.512 |
| 11 | 15.0 | 1.365 | 1.586 |

Resistance to exogenous proteolysis

Assessment of the resistance of the elastase CLEC to the action of protease was also performed under identical conditions as described for thermolysin (Example 2). Activity of the soluble and CLEC enzyme, following incubation with protease, was assayed by hydrolysis of the nitroanilide substrate as described above (Table 14 and Figure 6).

TABLE 14 -

| Elastase Resistance to Proteolysis | | | |
|---|---|---|---|
| | | % Maximum Activity | |
| | **Time** | **CLEC** | **soluble Enzyme** |
| 1 | 0.0 | 100.0 | 100.0 |
| 2 | 10.0 | | 53.0 |
| 3 | 20.0 | | 32.0 |
| 4 | 30.0 | 101.0 | 18.0 |
| 5 | 45.0 | | 11.0 |
| 6 | 60.0 | 102.0 | 8.0 |
| 7 | 120.0 | 101.0 | 3.0 |
| 8 | 180.0 | 103.0 | 2.0 |

Example 4

Crystallization, cross-linking and lyophilization of esterase and assessment of characteristics of the resulting product.

Crystallization of esterase

As disclosed here, 30 mg/ml ammonium sulfate suspension of pig liver esterase (Fluka) was dissolved in 0.25 M calcium acetate pH 5.6 at room temperature. Esterase crystals were visible within several minutes following addition of the calcium acetate solution. The crystallization solution was allowed to stand at room temperature and crystallization was completed overnight. Crystals were recovered by centrifugation as previously described in Example 2.

Cross-linking of esterase crystals

As disclosed here, a 300 µl volume of esterase crystals were added to a 5 ml solution of 12.5% glutaraldehyde and 0.5 M sodium acetate pH 5.5. The crystals were crosslinked for one hour with mild agitation (stir plate). Following cross-linking the crystals were washed with three 15 ml volumes of 0.5 M calcium acetate pH 6.3. The esterase CLEC was lyophilized as previously described in Example 2.

Enzymatic activity of soluble and CLEC esterase

The catalytic activity of soluble and CLEC esterase was assayed spectrophotometrically by monitoring hydrolysis of the substrate p-nitrophenyl acetate (Fluka) (Table 15 and Figure 7). Cleavage was monitored by increasing absorbance at 400 nm. Initial substrate concentration was 0.001%. Enzyme concentration was 1 X $10^{-8}$M. CLEC or soluble enzyme was added to a 5 ml reaction volume containing substrate in 0.25 M calcium acetate pH 6.3. As described previously in example 2, CLEC enzyme was removed from the reaction mix by centrifugation prior to measuring absorbance.

TABLE 15 -

| **Esterase Activity** | | | |
|---|---|---|---|
| | | **Absorbance 400 nm** | |
| | **Time (min)** | **CLEC** | **Soluble Enzyme** |
| 1 | 0.0 | 0.000 | 0.000 |
| 2 | 0.5 | 0.770 | 0.252 |
| 3 | 1.0 | 0.128 | 0.297 |
| 4 | 2.0 | 0.208 | 0.337 |
| 5 | 3.0 | 0.260 | 0.346 |
| 6 | 5.0 | 0.324 | 0.353 |
| 7 | 7.0 | 0.353 | 0.359 |
| 8 | 10.0 | 0.369 | 0.368 |

Resistance to exogenous proteolysis

Assessment of the resistance of the esterase CLEC to the action of protease was also performed under identical conditions as described for thermolysin (Example 2). Activity of the soluble and CLEC enzyme, following incubation with protease, was assayed by hydrolysis of the substrate p-nitrophenyl acetate as described above (Table 16 and Figure 8).

TABLE 16 -

| **Esterase Resistance to Proteolysis** | | | |
|---|---|---|---|
| | | **% Maximum Activity** | |
| | **Time (min)** | **CLEC** | **Soluble Enzymes** |
| 1 | 0.0 | 100.0 | 100.0 |
| 2 | 10.0 | | 68.0 |
| 3 | 20.0 | | 47.0 |
| 4 | 30.0 | 99.0 | 25.0 |
| 5 | 45.0 | | 20.0 |
| 6 | 60.0 | 97.0 | 16.0 |
| 7 | 120.0 | 94.0 | 10.0 |
| 8 | 180.0 | 91.0 | 6.0 |

Example 5

Crystallization, cross-linking and lyophilization of lipase and assessment of characteristics of the resulting product.

Crystallization of lipase

As disclosed here, the enzyme lipase (Geotrichum candidum) was crystallized by vapor diffusion from an aqueous solution of 20 mg/ml protein in 50 mM Tris pH 7 containing 8% ammonium sulfate. Bipyrimidal crystals were visible after 20 to 30 days incubation at room temperature. Crystals were recovered by centrifugation, as previously described in Example 2.

Cross-linking of lipase crystals

As disclosed here, lipase crystals were added to a solution of 12.5% glutaraldehyde and 50 mM Tris pH 5.6. The crystals were crosslinked for one hour. Following cross-linking the crystals were washed with three 15 ml volumes of 50 mM Tris pH 7.0. The lipase CLEC was lyophilized, as previously described in Example 2.

Enzymatic activity of soluble and CLEC lipase

The catalytic activity of soluble and CLEC lipase was assayed spectrophotometrically by monitoring hydrolysis of the substrate p-nitrophenyl acetate (Table 17 and Figure 9). Cleavage was monitored by increasing absorbance at 400 nm. Initial substrate concentration was 0.005%. Enzyme concentration was 1.5 X $10^{-8}$M. CLEC or soluble enzyme was added to a 5 ml reaction volume containing substrate in 0.2 M Tris pH 7.0 at room temperature. As described previously in Example 2, CLEC enzyme was removed from the reaction mix by centrifugation prior to measuring absorbance.

TABLE 17 -

| **Lipase Activity** | | | |
|---|---|---|---|
| | | **Absorbance 400 nm** | |
| | **Time (min)** | **CLEC** | **Soluble Enzyme** |
| 1 | 0.0 | 0.000 | 0.000 |
| 2 | 1.0 | 0.013 | 0.021 |
| 3 | 5.0 | 0.094 | 0.116 |
| 4 | 10.0 | 0.164 | 0.186 |
| 5 | 15.0 | 0.248 | 0.258 |
| 6 | 30.0 | 0.346 | 0.357 |
| 7 | 45.0 | 0.407 | 0.420 |
| 8 | 60.0 | 0.461 | 0.459 |
| 9 | 90.0 | 0.497 | 0.502 |

Example 6

Crystallization, cross-linking and lyophilization of lysozyme and assessment of characteristics of the resulting product

Crystallization of lysozyme

Following the method of Blake, C.C.F. et al., Nature 196:1173 (1962) 200 mg of lyophilized hen egg white lysozyme (Boehringer Mannheim) was dissolved in 2.5 ml of 0.04 M sodium acetate buffer pH 4.7 at room temperature. Following solvation of the protein, 2.5 ml of 10% sodium chloride were added to the lysozyme solution dropwise with stirring. The crystallization solution was allowed to stand overnight at room temperature, and crystallization was completed in forty-eight hours. Crystals were recovered by centrifugation, as previously described in Example 2.

Cross-linking of lysozyme crystals

As described here, a 500 µl volume of lysozyme crystals was added to 10 ml of 24% glutaraldehyde and 50 mM Tris pH 5.6 containing 20% sodium chloride. The crystals were crosslinked for 20 minutes with mild agitation (stir plate). Following cross-linking the crystals were washed with three 50 ml volumes of 20 mM calcium acetate and 50 mM potassium chloride pH 5.3. The lysozyme CLEC was lyophilized, as previously described in Example 2.

Enzymatic activity of soluble and CLEC lysozvme

The catalytic activity of soluble and CLEC lysozyme was assayed by measuring the rate of hydrolysis of the substrate 4-methylumbelliferyl N-acetyl-chitrioside (Fluka) (Yang, Y. and Hamaguchi, K. J. Biochem. 8:1003-1014 (1980) (Table 18 and Figure 10). The release of 4-methylumbelliferone was followed fluorimetrically (Perkin Elmer Model LS-50). Initial substrate concentration was 1.42 X $10^{-3}$. Enzyme concentration was 3 X $10^{-7}$. CLEC or soluble enzyme was added to a 2 ml reaction volume containing substrate in 20 mM calcium acetate and 50 mM potassium chloride pH 5.3 at 42°C. The amount of 4-methylumelliferone was determined fluorimetrically by measuring fluorescence intensities at 450 nm with excitation at 360 nm. Slit width for both excitation and emission was 10 mm. As described previously in Example 2, CLEC enzyme was removed from the reaction mix by centrifugation prior to measuring fluorescence.

TABLE 18 -

| Lysozyme Activity | | | |
|---|---|---|---|
| | | Fluorescence | |
| | Time (min) | CLEC | Soluble Enzyme |
| 1 | 0.000 | 0.000 | 0.000 |
| 2 | 10.000 | 4.400 | 18.900 |
| 3 | 30.000 | 10.500 | 29.400 |
| 4 | 60.000 | 27.500 | 44.800 |
| 5 | 90.000 | 33.800 | 51.700 |
| 6 | 120.000 | 45.900 | 59.800 |

Example 7

Crystallization, cross-linking and lyophilization of asparaginase and assessment of characteristics of the resulting product.

Crystallization of asparaginase

As a modification of the procedure described by Grabner et al. [U.S. Patent 3,664,926 (1972)] 25 mg of lyophilized asparaginase (Worthington) were dissolved in 500 µl of 50 mM sodium phosphate buffer pH 7.2. The solution was cooled to 4°C and the pH adjusted to 5.0 with 1 M acetic acid. Cold (-20°C) ethanol was then added dropwise to the asparaginase solution to a final concentration of 33%. The solution was incubated at 4°C. Crystallization was completed in forty-eight hours. Crystals were recovered by centrifugation as previously described.

Cross-linking of asparaginase crystals

As disclosed here, asparaginase crystals were crosslinked in a solution of 7.5% glutaraldehyde in 50 mM sodium phosphate buffer pH 5.6. Following crosslinking the crystals were washed with five 15 ml volumes of 50 mM tris pH 7.0. The asparaginase CLECs were lyophilized, as previously described in Example 2.

Enzymatic activity of soluble and CLEC asparaginase

The catalytic activity of soluble and CLEC asparaginase was assayed spectrophotometrically by measuring evolution of ammonium ion in the coupled enzymatic reaction described below (all reagents were purchased from Boehringer Mannheim) (Table 19 and Figure 11).

```
                    asparaginase
L-asparagine  ---------------->  asparate + NH4+

                                        glutamate dehydrogenase
NH4+ + NADH + α ketoglutarate  ------------------------> glutamic acid + NAI
```

Oxidation of NADH was measured by decreasing absorbance at 340 nm. Initial NADH concentration was 1.4 mg/ml. Asparagine concentration was $10^{-3}$M. Alpha ketoglutarate concentration was $10^{-4}$M. Glutamate dehydrogenase concentration was $10^{-7}$M. Asparaginase concentration was $2.3 \times 10^{-8}$M. As described previously in Example 2, CLEC enzyme was removed from the reaction mix by centrifugation prior to measuring absorbance.

TABLE 19 -

| Asparaginase Activity | | | |
|---|---|---|---|
| | | Absorbance 340 nm | |
| | Time (min) | CLEC | Soluble Enzyme |
| 1 | 0.0 | 1.000 | 1.000 |
| 2 | 1.0 | 0.867 | 0.825 |
| 3 | 3.0 | 0.739 | 0.684 |
| 4 | 5.0 | 0.603 | 0.538 |
| 5 | 10.0 | 0.502 | 0.406 |
| 6 | 15.0 | 0.449 | 0.338 |
| 7 | 30.0 | 0.328 | 0.199 |
| 8 | 45.0 | 0.211 | 0.187 |

## Claims

**Claims for the following Contracting States : AT, BE, CH, DE, DK, FR, GB, GR, IT, LI, LU, NL, SE**

**1.** An enzyme crystal crosslinked with a multifunctional crosslinking agent, said crosslinked enzyme crystal having resistance to exogenous Pronase™ proteolysis, wherein said enzyme is selected from the group consisting of pyridoxal phosphate enzymes, metalloenzymes, CoA-requiring enzymes and sulfurases (PAPS).

**2.** An enzyme crystal crosslinked with a multifunctional crosslinking agent, said crosslinked enzyme crystal having resistance to exogenous Pronase™ proteolysis, wherein said enzyme is selected from the group consisting of thermolysin, nitrilase, aminocyclase and hydantoinase.

**3.** The crosslinked enzyme crystal according to claim 2, wherein said enzyme is thermolysin.

**4.** An enzyme crystal crosslinked with a multifunctional crosslinking agent, said crosslinked enzyme crystal having resistance to exogenous Pronase™ proteolysis, wherein said enzyme is selected from the group consisting of elastase, aminopeptidase, asparaginase, glutaminase, arginase, urease, alkaline phosphatase, lipase, preferably phospholipase, β-lactamase, ACL hydrolase, L-ACT hydrolase, arylsulfatase, glycosidase, preferably β-galactosidase and β-fructosidase, penicillin acylase, amidase, preferably penicillin amidase, amino acid esterase, 5'-phosphodiesterase, nuclease and creatine deiminase.

**5.** The crosslinked enzyme crystal according to claim 4, wherein said enzyme is elastase.

**6.** The crosslinked enzyme crystal according to claim 4, wherein said enzyme is asparaginase.

**7.** The crosslinked enzyme crystal according to claim 4, wherein said enzyme is lipase.

8. An enzyme crystal crosslinked with a multifunctional crosslinking agent, said crosslinked enzyme crystal having resistance to exogenous Pronase™ proteolysis, wherein said enzyme is selected from the group consisting of amino acid dehydrogenase, formate dehydrogenase, lactate dehydrogenase, glutamate dehydrogenase, hydroxysteroid dehydrogenase, glucose-6-pyruvate dehydrogenase, succinate dehydrogenase, glucose dehydrogenase, catalase, superoxide dismutase, peroxidase, luciferase, tyrosinase, flavoenzyme, nitrate/nitrite reductase, oxidoreductase (NAD/P), oxidase and lyase.

9. The crosslinked enzyme crystal according to claim 8, wherein said oxidase is alcohol oxidase, glucose oxidase, bilirubin oxidase, lactate oxidase, cholesterol oxidase or L-amino oxidase, and/or said lyase is aldolase, L-aspartate 4-decarboxylase, phenylalanine ammonium lyase, carbonic anhydrase, nitrite hydratase, L-aspartase, fumarase or heparinase.

10. An enzyme crystal crosslinked with a multifunctional crosslinking agent, said crosslinked enzyme crystal having resistance to exogenous Pronase™ proteolysis, wherein said enzyme is selected from the group consisting of kinase (ATP), preferably creatine kinase, transaminase, glycosyl transferase and methyl transferase (SAM).

11. An enzyme crystal crosslinked with a multifunctional crosslinking agent, said crosslinked enzyme crystal having resistance to exogenous Pronase™ proteolysis, wherein said enzyme is selected from the group consisting of L-tryptophan synthetase and strictodine synthetase.

12. An enzyme crystal crosslinked with a multifunctional crosslinking agent, said crosslinked enzyme crystal having resistance to exogenous Pronase™ proteolysis, wherein said enzyme is esterase.

13. The crosslinked enzyme crystal according to any one of claims 1 to 12, wherein said crystal is a microcrystal having cross-section of approximately $10^{-1}$ mm.

14. The crosslinked enzyme crystal according to any one of claims 2, 4 or 12, said crosslinked enzyme crystal retaining at least 91% of its initial activity after incubation for three hours in the presence of a concentration of Pronase™ that causes the soluble uncrosslinked form of the enzyme that is crystallized to form said enzyme crystal that is crosslinked to retain at most 6% of its initial activity under the same conditions, wherein said enzyme is selected from the group consisting of esterase, elastase and thermolysin.

15. The crosslinked enzyme crystal according to any one of claims 1 to 14, wherein the crosslinker is glutaraldehyde.

16. A device comprising a crosslinked enzyme crystal according to any one of claims 1 to 15, and a retaining means for said crosslinked enzyme crystal, said retaining means consisting of a material which allows contact between said crosslinked enzyme crystal and a substrate upon which the enzyme acts, the substrate being present in a fluid.

17. A biosensor for detecting an analyte of interest in a fluid, comprising:

a) a crosslinked enzyme crystal according to any one of claims 1 to 15, wherein the enzyme present acts on the analyte of interest or on a reactant in a reaction in which the analyte of interest participates; and
b) a retaining means for said crosslinked enzyme crystal, the retaining means consisting of a material which allows contact between said crosslinked enzyme crystal and a fluid, said fluid containing either (1) the analyte upon which the enzyme acts or (2) a reactant in a reaction in which the analyte participates.

18. The biosensor according to claim 17, said biosensor further comprising a detecting means.

19. The use of a biosensor according to claim 17 or 18 for detecting an analyte of interest in a fluid, wherein said analyte of interest is selected from the group consisting of: glucose, creatinine, urea, lactate, glucose-6-phosphate, sucrose, ATP, ethanol, acetic acid, formic acid, cholesterol, uric acid, methotrexate, carbon dioxide, amino acids, phosphates, penicillins, nitrates, nitrites, sulphates and succinate.

20. The biosensor according to claim 17, said crosslinked enzyme crystal being a luciferase crystal, wherein the luciferase acts on the analyte of interest or on a product of a reaction in which the analyte of interest participates and said retaining means consists of a material which allows contact between said crosslinked luciferase crystal and a fluid, said fluid containing either (1) the analyte upon which the luciferase acts or (2) the product of a reaction in which the analyte participates.

**21.** An extracorporeal device for use in altering a component of a fluid comprising:

a) a crosslinked enzyme crystal according to any one of claims 1 to 15, wherein the enzyme present acts on the component or on a reactant in a reaction in which the component participates; and
b) a retaining means for said crosslinked enzyme crystal, said retaining means consisting of a material which allows contact between said crosslinked enzyme crystal and a fluid, said fluid containing either (1) the component upon which the enzyme acts or (2) the product of a reaction in which the component participates.

**22.** The use of an extracorporeal device according to claim 21 for altering a component of a fluid, wherein said component is selected from the group consisting of asparagine, heparin, bilirubin, methotrexate, amino acids, urea and ammonia.

**23.** A device for use in producing a selected product, comprising an enzyme in the form of a crosslinked enzyme crystal according to any one of claims 1 to 15 and a retaining means for said crosslinked enzyme crystal.

**24.** A method of producing aspartame comprising the steps of:

a) combining two peptides and a crosslinked thermolysin crystal according to claim 2, the first peptide having the formula. Z-L-Asp and the second peptide having the formula L-Phe-OMe, under conditions appropriate for condensation of the two peptides through action of said crosslinked thermolysin crystal, thereby producing a partially protected dipeptide of the formula Z-L-Asp-L-Phe-OMe, wherein Z represents a benzyloxycarbonyl group, and
b) removing said benzyloxycarbonyl group from said dipeptide, thereby producing aspartame.

**Claims for the following Contracting State : ES**

**1.** A method for the preparation of an enzyme crystal crosslinked with a multifunctional crosslinking agent, said crosslinked enzyme crystal having resistance to exogenous Pronase™ proteolysis, wherein said enzyme is selected from the group consisting of pyridoxal phosphate enzymes, metalloenzymes, CoA-requiring enzymes and sulfurases (PAPS), comprising crystallisation of the enzyme followed by cross-linking the crystal lattice with a multifunctional reagent.

**2.** A method for the preparation of an enzyme crystal crosslinked with a multifunctional crosslinking agent, said crosslinked enzyme crystal having resistance to exogenous Pronase™ proteclysis, wherein said enzyme is selected from the group consisting of thermolysin, nitrilase,
aminocyclase and hydantoinase, comprising crystallisation of the enzyme followed by cross-linking the crystal latticewith a multifunctional reagent.

**3.** The method according to claim 2, wherein said enzyme is thermolysin.

**4.** A method for the preparation of an enzyme crystal crosslinked with a multifunctional crosslinking agent, said crosslinked enzyme crystal having resistance to exogenous Pronase™ proteolysis, wherein said enzyme is selected from the group consisting of elastase, aminopeptidase, asparaginase, glutaminase, arginase, urease, alkaline phosphatase, lipase, preferably phospholipase, β-lactamase, ACL hydrolase, L-ACT hydrolase, arylsulfatase, glycosidase, preferably β-galactosidase and β-fructosidase, penicillin acylase, amidase, preferably penicillin amidase, amino acid esterase, 5'-phosphodiesterase, nuclease and creatine deiminase, comprising crystallisation of the enzyme followed by cross-linking the crystal lattice with a multifunctional reagent.

**5.** The method according to claim 4, wherein said enzyme is elastase.

**6.** The method according to claim 4, wherein said enzyme is asparaginase.

**7.** The method according to claim 4, wherein said enzyme is lipase.

**8.** A method for the preparation of an enzyme crystal crosslinked with a (multifunctional crosslinking agent, said crosslinked enzyme crystal having resistance to exogenous Pronase™ proteolysis, wherein said enzyme is selected from the group consisting of amino acid dehydrogenase, formate dehydrogenase, lactate dehydrogenase, glutamate dehydrogenase, hydroxysteroid dehydrogenase, glucose-6-pyzuvate dehydrogenase, succinate dehydrogenase, glucose dehydrogenase, catalase, superoxide dismutase, peroxidase, luciferase, tyrosinase, flavoenzyme, nitrate/nitrite reductase, oxidoreductase (NAD/P), oxidase and lyase, comprising crystallisation of the enzyme followed by cross-linking the crystal lattice with a multifunctional reagent.

**9.** The method according to claim 8, wherein said oxidase is alcohol oxidase, glucose oxidase, bilirubin oxidase, lactate oxidase, cholesterol oxidase or L-amino oxidase, and/or said lyase is aldolase, L-aspartate 4-decarboxylase, phenylalanine ammonium lyase, carbonic anhydrase, nitrile hydratase, L-aspartase, fumarase or heparinase.

**10.** A method for the preparation of an enzyme crystal crosslinked with a multifunctional crosslinking agent, said crosslinked enzyme crystal having resistance to exogenous Pronase™ proteolysis, wherein said enzyme is selected from the group consisting of kinase (ATP), preferably creatine kinase, transaminase, glycosyl transferase and methyl transferase (SAM), comprising crystallisation of the enzyme followed by cross-linking the crystal latticewith a multifunctional reagent.

**11.** A method for the preparation of an enzyme crystal crosslinked with a multifunctional crosslinking agent, said crosslinked enzyme crystal having resistance to exogenous Pronase™ proteolysis, wherein said enzyme is selected from the group consisting of L-tryptophan synthetase and strictodine synthetase, comprising crystallisation of the enzyme followed by cross-linking the crystal lattice with a multifunctional reagent.

**12.** A method for the preparation of an enzyme crystal crosslinked with a multifunctional crosslinking agent, said crosslinked enzyme crystal having resistance to exogenous Pronase™ proteolysis, wherein said enzyme is esterase, comprising crystallisation of the enzyme followed by cross-linking the crystal lattice with a multifunctional reagent.

**13.** The method according to any one of claims 1 to 12, wherein said crystal is a microcrystal having cross-section of approximately $10^{-1}$ mm.

**14.** The method according to any one of claims 2, 4 or 12, said crosslinked enzyme crystal retaining at least 91% of its initial activity after incubation for three hours in the presence of a concentration of Pronase™ that causes the soluble uncrosslinked form of the enzyme that is crystallized to form said enzyme crystal that is crosslinked to retain at most 6% of its initial activity under the same conditions, wherein said enzyme is selected from the group consisting of esterase, elastase and thermolysin.

**15.** The method according to any one of claims 1 to 14, wherein the crosslinker is glutaraldehyde.

**16.** A device comprising a crosslinked enzyme crystal according to any one of claims 1 to 15, and a retaining means for said crosslinked enzyme crystal, said retaining means consisting of a material which allows contact between said crosslinked enzyme crystal and a substrate upon which the enzyme acts, the substrate being present in a fluid.

**17.** A biosensor for detecting an analyte of interest in a fluid, comprising:

a) a crosslinked enzyme crystal according to any one of claims 1 to 15, wherein the enzyme present acts on the analyte of interest or on a reactant in a reaction in which the analyte of interest participates; and
b) a retaining means for said crosslinked enzyme crystal, the retaining means consisting of a material which allows contact between said crosslinked enzyme crystal and a fluid, said fluid containing either (1) the analyte upon which the enzyme acts or (2) a reactant in a reaction in which the analyte participates.

**18.** The biosensor according to claim 17, said biosensor further comprising a detecting means.

**19.** The use of a biosensor according to claim 17 or 18 for detecting an analyte of interest in a fluid, wherein said analyte of interest is selected from the group consisting of: glucose, creatinine, urea, lactate, glucose-6-phosphate, sucrose, ATP, ethanol, acetic acid, formic acid, cholesterol, uric acid, methotrexate, carbon dioxide, amino acids, phosphates, penicillins, nitrates, nitrites, sulphates and succinate.

**20.** The biosensor according to claim 17, said crosslinked enzyme crystal being a luciferase crystal, wherein the luciferase acts on the analyte of interest or on a product of a reaction in which the analyte of interest participates and said retaining means consists of a material which allows contact between said crosslinked luciferase crystal and a fluid, said fluid containing either (1) the analyte upon which the luciferase acts or (2) the product of a reaction in which the analyte participates.

**21.** An extracorporeal device for use in altering a component of a fluid comprising:

a) a crosslinked enzyme crystal according to any one of claims 1 to 15, wherein the enzyme present acts on the component or on a reactant in a reaction in which the component participates; and
b) a retaining means for said crosslinked enzyme crystal, said retaining means consisting of a material which allows contact between said crosslinked enzyme crystal and a fluid, said fluid containing either (1) the component upon which the enzyme acts or (2) the product of a reaction in which the component participates.

**22.** The use of an extracorporeal device according to claim 21 for altering a component of a fluid, wherein said component is selected from the group consisting of asparagine, heparin, bilirubin, methotrexate, amino acids, urea and ammonia.

**23.** A device for use in producing a selected product, comprising an enzyme in the form of a crosslinked enzyme crystal according to any one of claims 1 to 15 and a retaining means for said crosslinked enzyme crystal.

**24.** A method of producing aspartame comprising the steps of:

a) combining two peptides and *a* crosslinked thermolysin crystal according to claim 2, the first peptide having the formula Z-L-Asp and the second peptide having the formula L-Phe-OMe, under conditions appropriate for condensation of the two peptides through action of said crosslinked thermolysin crystal, thereby producing a partially protected dipeptide of the formula Z-L-Asp-L-Phe-OMe, wherein Z represents a benzyloxycarbonyl group, and
b) removing said benzyloxycarbonyl group from said dipeptide, thereby producing aspartame.

**Patentansprüche**

**Patentansprüche für folgende Vertragsstaaten : AT, BE, CH, DE, DK, FR, GB, GR, IT, LI, LU, NL, SE**

**1.** Enzymkristall, der mit einem multifunktionalen Vernetzungsmittel vernetzt ist, wobei der vernetzte Enzymkristall eine Resistenz gegen exogene Pronase™-Proteolyse hat, wobei das Enzym ausgewählt ist aus der Gruppe bestehend aus Pyridoxalphosphatenzymen, Metallenzymen, Enzymen, die CoA benötigen, und Sulfurasen (PAPS).

**2.** Enzymkristall, der mit einem multifunktionalen Vernetzungsmittel vernetzt ist, wobei der vernetzte Enzymkristall eine Resistenz gegen exogene Pronase™-Proteolyse hat, wobei das Enzym ausgewählt ist aus der Gruppe bestehend aus Thermolysin, Nitrilase, Aminocyclase und Hydantoinase.

**3.** Vernetzter Enzymkristall nach Anspruch 2, wobei das Enzym Thermolysin ist.

**4.** Enzymkristall, der mit einem multifunktionalen Vernetzungsmittel vernetzt ist, wobei der vernetzte Enzymkristall eine Resistenz gegen exogene Pronase™-Proteolyse hat, wobei das Enzym ausgewählt ist aus der Gruppe bestehend aus Elastase, Aminopeptidase, Asparaginase, Glutaminase, Arginase, Urease, alkalischer Phosphatase, Lipase, vorzugsweise Phospholipase, β-Lactamase, ACL-Hydrolase, L-ACT-Hydrolase, Arylsulfatase, Glycosidase, vorzugsweise β-Galactosidase und β-Fructosidase, Penicillinacylase, Amidase, vorzugsweise Penicillinamidase, Aminosäureesterase, 5'-Phosphodiesterase, Nuclease und Creatindeiminase.

**5.** Vernetzter Enzymkristall nach Anspruch 4, wobei das Enzym Elastase ist.

**6.** Vernetzter Enzymkristall nach Anspruch 4, wobei das Enzym Asparaginase ist.

**7.** Vernetzter Enzymkristall nach Anspruch 4, wobei das Enzym Lipase ist.

**8.** Enzymkristall, der mit einem multifunktionalen Vernetzungsmittel vernetzt ist, wobei der vernetzte Enzymkristall eine Resistenz gegen exogene Pronase™-Proteolyse hat, wobei das Enzym ausgewählt ist aus der Gruppe bestehend aus Aminosäuredehydrogenase, Formiatdehydrogenase, Lactatdehydrogenase, Glutamatdehydrogenase, Hydroxysteroiddehydrogenase, Glucose-6-Pyruvatdehydrogenase, Succinatdehydrogenase, Glucosedehydrogenase, Katalase, Superoxiddismutase, Peroxidase, Luciferase, Tyrosinase, Flavoenzym, Nitrat-/Nitritreductase, Oxidoreductase (NAD/P), Oxidase und Lyase.

**9.** Vernetzter Enzymkristall nach Anspruch 8, wobei die Oxidase Alkoholoxidase, Glucoseoxidase, Bilirubinoxidase, Lactatoxidase, Cholesteroloxidase oder L-Aminooxidase, und/oder die Lyase Aldolase, L-Asparatat-4-decarboxylase, Phenylalaninammoniumlyase, Carboanhydrase, Nitrilhydratase, L-Aspartase, Fumarase oder Heparinase ist.

**10.** Enzymkristall, vernetzt mit einem multifunktionalen Vernetzungsmittel, wobei der vernetzte Enzymkristall eine Resistenz gegen exogene Pronase™-Proteolyse hat, wobei das Enzym ausgewählt ist aus der Gruppe bestehend aus Kinase (ATP), vorzugsweise Kreatinkinase, Transaminase, Glycosyltransferase und Methyltransferase (SAM).

**11.** Enzymkristall, vernetzt mit einem multifunktionalen Vernetzungsmittel, wobei der vernetzte Enzymkristall eine Resistenz gegen exogene Pronase™-Proteolyse hat, wobei das Enzym ausgewählt ist aus der Gruppe bestehend aus L-Tryptophansynthetase und Strictodinsynthetase.

**12.** Enzymkristall, vernetzt mit einem multifunktionalen Vernetzungsmittel, wobei der vernetzte Enzymkristall eine Resistenz gegen exogene Pronase™-Proteolyse hat, wobei das Enzym Esterase ist.

**13.** Vernetzter Enzymkristall nach einem der Ansprüche 1 bis 12, wobei der Kristall ein Mikrokristall mit einem Querschnitt von ungefähr $10^{-1}$ mm ist.

**14.** Vernetzter Enzymkristall nach einem der Ansprüche 2, 4 oder 12, wobei der vernetzte Enzymkristall mindestens 91% seiner anfänglichen Aktivität nach einer Inkubation von 3 Stunden in Gegenwart einer Konzentration von Pronase™ behält, die bewirkt, dass die lösliche, nicht vernetzte Form des Enzyms, das kristallisiert wird, um den Enzymkristall zu bilden, der vernetzt wird, höchstens 6% ihrer anfänglichen Aktivität unter den gleichen Bedingungen behält, wobei das Enzym ausgewählt ist aus der Gruppe bestehend aus Esterase, Elastase und Thermolysin.

**15.** Vernetzter Enzymkristall nach einem der Ansprüche 1 bis 14, wobei der Quervernetzer Glutaraldehyd ist.

**16.** Vorrichtung, umfassend einen vernetzten Enzymkristall nach einem der Ansprüche 1 bis 15 und ein Rückhaltemittel für den vernetzten Enzymkristall, wobei das Rückhaltemittel aus einem Material besteht, das einen Kontakt zwischen dem vernetzten Enzymkristall und dem Substrat erlaubt, auf das das Enzym wirkt, wobei das Substrat in einer Flüssigkeit vorhanden ist.

**17.** Biosensor zum Nachweis eines Analyten von Interesse in einer Flüssigkeit, umfassend:

a) einen vernetzten Enzymkristall nach einem der Ansprüche 1 bis 15, wobei das vorhandene Enzym auf den Analyten von Interesse oder auf einen Reaktionspartner in einer Reaktion wirkt, an der der Analyt von Interesse beteiligt ist; und
b) ein Rückhaltemittel für den vernetzten Enzymkristall, wobei das Rückhaltemittel aus einem Material besteht, das den Kontakt zwischen dem quervernetzen Enzymkristall und einer Flüssigkeit erlaubt, wobei die Flüssigkeit entweder (1) den Analyten, auf den das Enzym wirkt, oder (2) einen Reaktionspartner in einer Reaktion enthält, an der der Analyt beteiligt ist.

**18.** Biosensor nach Anspruch 17, wobei der Biosensor außerdem ein Nachweismittel umfasst.

**19.** Verwendung eines Biosensors nach Anspruch 17 oder 18 zum Nachweis eines Analyts von Interesse in einer Flüssigkeit, wobei der Analyt von Interesse ausgewählt ist aus der Gruppe bestehend aus: Glucose, Kreatinin, Harnstoff, Lactat, Glucose-6-phosphat, Saccharose, ATP, Ethanol, Essigsäure, Ameisensäure, Cholesterin, Harnsäure, Methotrexat, Kohlendioxid, Aminosäuren, Phosphaten, Penicillinen, Nitraten, Nitriten, Sulphaten und Succinat.

**20.** Biosensor nach Anspruch 17, wobei der vernetzte Enzymkristall ein Luciferasekristall ist, wobei die Luciferase auf den Analyten von Interesse oder auf ein Produkt einer Reaktion wirkt, an dem der Analyt von Interesse beteiligt ist, und wobei das Rückhaltemittel aus einem Material besteht, das den Kontakt zwischen dem vernetzten Luciferasekristall und einer Flüssigkeit erlaubt, wobei die Flüssigkeit entweder (1) den Analyten, auf den die Luciferase wirkt, oder (2) das Produkt einer Reaktion enthält, an der der Analyt beteiligt ist.

**21.** Extrakorporale Vorrichtung zur Verwendung in der Änderung eines Bestandteils einer Flüssigkeit umfassend:

a) einen vernetzten Enzymkristall nach einem der Ansprüche 1 bis 15, wobei das vorliegende Enzym auf den Bestandteil oder auf einen Partner einer Reaktion wirkt, an der der Bestandteil beteiligt ist; und
b) ein Rückhaltemittel für den vernetzten Enzymkristall, wobei das Rückhaltemittel aus einem Material besteht, das den Kontakt zwischen dem quervernetzen Enzymkristall und einer Flüssigkeit erlaubt, wobei die Flüssigkeit entweder (1) den Bestandteil, auf den das Enzym wirkt oder (2) das Produkt einer Reaktion enthält, an der der Bestandteil beteiligt ist.

**22.** Verwendung einer extrakorporalen Vorrichtung nach Anspruch 21 zur Änderung eines Bestandteils einer Flüssigkeit, wobei der Bestandteil ausgewählt ist aus der Gruppe bestehend aus Asparagin, Heparin, Bilirubin, Methotrexat, Aminosäuren, Harnstoff und Ammoniak.

**23.** Vorrichtung zur Verwendung bei der Herstellung eines ausgewählten Produkts, umfassend ein Enzym in der Form eines vernetzten Enzymkristalls nach einem der Ansprüche 1 bis 15 und ein Rückhaltemittel für den vernetzten Enzymkristall.

**24.** Verfahren zur Herstellung von Aspartam, umfassend die Schritte:

a) Kombinieren von zwei Peptiden und einem vernetzten Thermolysinkristall nach Anspruch 2, wobei das erste Peptid die Formel Z-L-Asp und das zweite Peptid die Formel L-Phe-OMe hat, unter Bedingungen, die geeignet sind zur Kondensation der zwei Peptide durch die Wirkung des vernetzten Thermolysinkristalls, wobei ein teilweise geschütztes Dipeptid der Formel Z-L-Asp-L-Phe-OMe hergestellt wird, wobei Z eine Benzyloxycarbonylgruppe ist, und
b) Entfernen der Benzyloxycarbonylgruppe aus dem Dipeptid, wobei Aspartam hergestellt wird.

**Patentansprüche für folgenden Vertragsstaat : ES**

**1.** Verfahren zur Herstellung eines Enzymkristalls, der mit einem multifunktionalen Vernetzungsmittel vernetzt ist, wobei der vernetzte Enzymkristall eine Resistenz gegen exogene Pronase™-Proteolyse hat, wobei das Enzym ausgewählt ist aus der Gruppe bestehend aus Pyridoxalphosphatenzymen, Metallenzymen, Enzymen, die CoA benötigen, und Sulfurasen (PAPS), umfassend die Kristallisierung des Enzyms mit anschließender Vernetzung des Kristallgitters mit einem multifunktionalen Reagens.

**2.** Verfahren zur Herstellung eines Enzymkristalls, der mit einem multifunktionalen Vernetzungsmittel vernetzt ist, wobei der vernetzte Enzymkristall eine Resistenz gegen exogene Pronase™-Proteolyse hat, wobei das Enzym ausgewählt ist aus der Gruppe bestehend aus Thermolysin, Nitrilase, Aminocyclase und Hydantoinase, umfassend die Kristallisierung des Enzyms mit anschließender Vernetzung des Kristallgitters mit einem multifunktionalen Reagens.

**3.** Verfahren nach Anspruch 2, wobei das Enzym Thermolysin ist.

**4.** Verfahren zur Herstellung eines Enzymkristalls, der mit einem multifunktionalen Vernetzungsmittel vernetzt ist, wobei der vernetzte Enzymkristall eine Resistenz gegen exogene Pronase™-Proteolyse hat, wobei das Enzym ausgewählt ist aus der Gruppe bestehend aus Elastase, Aminopeptidase, Asparaginase, Glutaminase, Arginase, Urease, alkalischer Phosphatase, Lipase, vorzugsweise Phospholipase, β-Lactamase, ACL-Hydrolase, L-ACT-Hydrolase, Arylsulfatase, Glycosidase, vorzugsweise β-Galactosidase und β-Fructosidase, Penicillinacylase, Amidase, vorzugsweise Penicillinamidase, Aminosäureesterase, 5'-Phosphodiesterase, Nuclease und Creatindeiminase, umfassend die Kristallisierung des Enzyms mit anschließender Vernetzung des Kristallgitters mit einem multifunktionalen Reagens.

**5.** Verfahren nach Anspruch 4, wobei das Enzym Elastase ist.

**6.** Verfahren nach Anspruch 4, wobei das Enzym Asparaginase ist.

**7.** Verfahren nach Anspruch 4, wobei das Enzym Lipase ist.

**8.** Verfahren zur Herstellung eines Enzymkristalls, der mit einem multifunktionalen Vernetzungsmittel vernetzt ist, wobei der vernetzte Enzymkristall eine Resistenz gegen exogene Pronase™-Proteolyse hat, wobei das Enzym ausgewählt ist aus der Gruppe bestehend aus Aminosäuredehydrogenase, Formiatdehydrogenase, Lactatdehydrogenase, Glutamatdehydrogenase, Hydroxysteroiddehydrogenase, Glucose-6-Pyruvatdehydrogenase, Succinatdehydrogenase, Glucosedehydrogenase, Katalase, Superoxiddismutase, Peroxidase, Luciferase, Tyrosinase, Flavoenzym, Nitrat-/Nitritreductase, Oxidoreductase (NAD/P), Oxidase und Lyase, umfassend die Kristallisierung des Enzyms mit anschließender Vernetzung des Kristallgitters mit einem multifunktionalen Reagens.

**9.** Verfahren nach Anspruch 8, wobei die Oxidase Alkoholoxidase, Glucoseoxidase, Bilirubinoxidase, Lactatoxidase, Cholesteroloxidase oder L-Aminooxidase, und/oder die Lyase Aldolase, L-Asparatat-4-decarboxylase, Phenylalaninammoniumlyase, Carboanhydrase, Nitrilhydratase, L-Aspartase, Fumarase oder Heparinase ist.

**10.** Verfahren zur Herstellung eines Enzymkristalls, vernetzt mit einem multifunktionalen Vernetzungsmittel, wobei der vernetzte Enzymkristall eine Resistenz gegen exogene Pronase™-Proteolyse hat, wobei das Enzym ausgewählt ist aus der Gruppe bestehend aus Kinase (ATP), vorzugsweise Kreatinkinase, Transaminase, Glycosyltransferase und Methyltransferase (SAM), umfassend die Kristallisierung des Enzyms mit anschließender Vernetzung des Kristallgitters mit einem multifunktionalen Reagens.

**11.** Verfahren zur Herstellung eines Enzymkristalls, vernetzt mit einem multifunktionalen Vernetzungsmittel, wobei der vernetzte Enzymkristall eine Resistenz gegen exogene Pronase™-Proteolyse hat, wobei das Enzym ausgewählt ist aus der Gruppe bestehend aus L-Tryptophansynthetase und Strictodinsynthetase, umfassend die Kristallisierung des Enzyms mit anschließender Vernetzung des Kristallgitters mit einem multifunktionalen Reagens.

**12.** Verfahren zur Herstellung eines Enzymkristalls, vernetzt mit einem multifunktionalen Vernetzungsmittel, wobei der vernetzte Enzymkristall eine Resistenz gegen exogene Pronase™-Proteolyse hat, wobei das Enzym Esterase ist, umfassend die Kristallisierung des Enzymes mit anschließender Vernetzung des Kristallgitters mit einem multifunktionalen Reagens.

**13.** Verfahren nach einem der Ansprüche 1 bis 12, wobei der Kristall ein Mikrokristall mit einem Querschnitt von ungefähr $10^{-1}$ mm ist.

**14.** Verfahren nach einem der Ansprüche 2, 4 oder 12, wobei der vernetzte Enzymkristall mindestens 91% seiner anfänglichen Aktivität nach einer Inkubation von 3 Stunden in Gegenwart einer Konzentration von Pronase™ behält, die bewirkt, dass die lösliche, nicht vernetzte Form des Enzyms, das kristallisiert wird, um den Enzymkristall zu bilden, der vernetzt wird, höchstens 6% ihrer anfänglichen Aktivität unter den gleichen Bedingungen behält, wobei das Enzym ausgewählt ist aus der Gruppe bestehend aus Esterase, Elastase und Thermolysin.

**15.** Verfahren nach einem der Ansprüche 1 bis 14, wobei der Quervernetzer Glutaraldehyd ist.

**16.** Vorrichtung, umfassend einen vernetzten Enzymkristall nach einem der Ansprüche 1 bis 15 und ein Rückhaltemittel für den vernetzten Enzymkristall, wobei das Rückhaltemittel aus einem Material besteht, das einen Kontakt zwischen dem vernetzten Enzymkristall und dem Substrat erlaubt, auf das das Enzym wirkt, wobei das Substrat in einer Flüssigkeit vorhanden ist.

**17.** Biosensor zum Nachweis eines Analyten von Interesse in einer Flüssigkeit, umfassend:

a) einen vernetzten Enzymkristall nach einem der Ansprüche 1 bis 15, wobei das vorhandene Enzym auf den Analyten von Interesse oder auf einen Reaktionspartner in einer Reaktion wirkt, an der der Analyt von Interesse beteiligt ist; und
b) ein Rückhaltemittel für den vernetzten Enzymkristall, wobei das Rückhaltemittel aus einem Material besteht, das den Kontakt zwischen dem quervernetzen Enzymkristall und einer Flüssigkeit erlaubt, wobei die Flüssigkeit entweder (1) den Analyten, auf den das Enzym wirkt, oder (2) einen Reaktionspartner in einer Reaktion enthält, an der der Analyt beteiligt ist.

**18.** Biosensor nach Anspruch 17, wobei der Biosensor außerdem ein Nachweismittel umfasst.

**19.** Verwendung eines Biosensors nach Anspruch 17 oder 18 zum Nachweis eines Analyts von Interesse in einer Flüssigkeit, wobei der Analyt von Interesse ausgewählt ist aus der Gruppe bestehend aus: Glucose, Kreatinin, Harnstoff, Lactat, Glucose-6-phosphat, Saccharose, ATP, Ethanol, Essigsäure, Ameisensäure, Cholesterin, Harnsäure, Methotrexat, Kohlendioxid, Aminosäuren, Phosphaten, Penicillinen, Nitraten, Nitriten, Sulphaten und Succinat.

**20.** Biosensor nach Anspruch 17, wobei der vernetzte Enzymkristall ein Luciferasekristall ist, wobei die Luciferase auf den Analyten von Interesse oder auf ein Produkt einer Reaktion wirkt, an dem der Analyt von Interesse beteiligt ist, und wobei das Rückhaltemittel aus einem Material besteht, das den Kontakt zwischen dem vernetzten Luciferasekristall und einer Flüssigkeit erlaubt, wobei die Flüssigkeit entweder (1) den Analyten, auf den die Luciferase wirkt, oder (2) das Produkt einer Reaktion enthält, an der der Analyt beteiligt ist.

**21.** Extrakorporale Vorrichtung zur Verwendung in der Änderung eines Bestandteils einer Flüssigkeit umfassend:

a) einen vernetzten Enzymkristall nach einem der Ansprüche 1 bis 15, wobei das vorliegende Enzym auf den Bestandteil oder auf einen Partner einer Reaktion wirkt, an der der Bestandteil beteiligt ist; und
b) ein Rückhaltemittel für den vernetzten Enzymkristall, wobei das Rückhaltemittel aus einem Material besteht, das den Kontakt zwischen dem quervernetzen Enzymkristall und einer Flüssigkeit erlaubt, wobei die Flüssigkeit entweder (1) den Bestandteil, auf den das Enzym wirkt, oder (2) das Produkt einer Reaktion enthält, an der der Bestandteil beteiligt ist.

**22.** Verwendung einer extrakorporalen Vorrichtung nach Anspruch 21 zur Änderung eines Bestandteils einer Flüssigkeit, wobei der Bestandteil ausgewählt ist aus der Gruppe bestehend aus Asparagin, Heparin, Bilirubin, Methotrexat, Aminosäuren, Harnstoff und Ammoniak.

**23.** Vorrichtung zur Verwendung bei der Herstellung eines ausgewählten Produkts, umfassend ein Enzym in der Form eines vernetzten Enzymkristalls nach einem der Ansprüche 1 bis 15 und ein Rückhaltemittel für den vernetzten Enzymkristall.

**24.** Verfahren zur Herstellung von Aspartam, umfassend die Schritte:

a) Kombinieren von zwei Peptiden und einem vernetzten Thermolysinkristall nach Anspruch 2, wobei das erste Peptid die Formel Z-L-Asp und das zweite Peptid die Formel L-Phe-OMe hat, unter Bedingungen, die geeignet sind zur Kondensation der zwei Peptide durch die Wirkung des vernetzten Thermolysinkristalls, wobei ein teilweise geschütztes Dipeptid der Formel Z-L-Asp-L-Phe-OMe hergestellt wird, wobei Z eine Benzyloxycarbonylgruppe ist, und
b) Entfernen der Benzyloxycarbonylgruppe aus dem Dipeptid, wobei Aspartam hergestellt wird.

## Revendications

### Revendications pour les Etats contractants suivants : AT, BE, CH, DE, DK, FR, GB, GR, IT, LI, LU, NL, SE

**1.** Cristal d'enzyme réticulé avec un agent réticulant multifonctionnel, ledit cristal d'enzyme réticulé ayant une résistance à la protéolyse à la Pronase™ exogène où ladite enzyme est sélectionnée dans le groupe consistant en enzymes de pyridoxal phosphate, métalloenzymes, enzymes nécessitant CoA et sulfurases (PAPS).

**2.** Cristal d'enzyme réticulé avec un agent réticulant multifonctionnel, ledit cristal d'enzyme réticulé ayant une résistance à la protéolyse à la Pronase™ exogène où ladite enzyme est sélectionnée dans le groupe consistant en thermolysine, nitrilase, aminocyclase et hydantoïnase.

**3.** Cristal d'enzyme réticulé selon la revendication 2, où ladite enzyme est la thermolysine.

**4.** Cristal d'enzyme réticulé avec un agent réticulant multifonctionnel, ledit cristal d'enzyme réticulé ayant une résistance à la protéolyse à la Pronase™ exogène, où ladite enzyme est sélectionnée dans le groupe consistant en élastase, aminopeptidase, asparaginase, glutaminase, arginase, uréase, phosphatase alcaline, lipase, de préférence phospholipase, β-lactamase, ACL hydrolase, L-ACT hydrolase, arylsulfatase, glycosidase, de préférence β-galactosidase et β-fructosidase, pénicilline acylase, amidase, de préférence pénicilline amidase, estérase d'acide aminé, 5'-phosphodiestérase, nucléase et créatine désiminase.

**5.** Cristal d'enzyme réticulé selon la revendication 4, où ladite enzyme est l'élastase.

**6.** Cristal d'enzyme réticulé selon la revendication 4, où ladite enzyme est l'asparaginase.

**7.** Cristal d'enzyme réticulé selon la revendication 4, où ladite enzyme est la lipase.

**8.** Cristal d'enzyme réticulé avec un agent réticulant multifonctionnel, ledit cristal d'enzyme réticulé ayant une résistance à la protéolyse à la Pronase™ exogène, où ladite enzyme est sélectionnée dans le groupe consistant en déshydrogénase d'acide aminé, formiate déshydrogénase, lactate déshydrogénase, glutamate déshydrogénase, hydroxystéroïde déshydrogénase, glucose-6-pyruvate déshydrogénase, succinate déshydrogénase, glucose déshydrogénase, catalase, superoxyde dismutase, peroxydase, luciférase, tyrosinase, flavoenzyme, nitrate/nitrite réductase, oxydoréductase (NAD/P), oxydase et lyase.

**9.** Cristal d'enzyme réticulé selon la revendication 8, où ladite oxydase est alcool oxydase, glucose oxydase, bilirubine oxydase, lactate oxydase, cholestérol oxydase ou oxydase d'acide L-aminé, et/ou ladite lyase est aldolase, L-aspartate 4-décarboxylase, phénylalanine ammonium lyase, anhydrase carbonique, nitrile hydratase, L-aspartase, fumarase ou héparinase.

**10.** Cristal d'enzyme réticulé avec un agent réticulant multifonctionnel, ledit cristal d'enzyme réticulé ayant une résistance à la protéolyse à la Pronase™ exogène, où ladite enzyme est sélectionnée dans le groupe consistant en kinase (ATP), de préférence créatine kinase, transaminase, glycosyl transférase et méthyl transférase (SAM).

**11.** Cristal d'enzyme réticulé avec un agent réticulant multifonctionnel, ledit cristal d'enzyme réticulé ayant une résistance à la protéolyse à la Pronase™ exogène, où ladite enzyme est sélectionnée dans le groupe consistant en L-tryptophane synthétase et strictodine synthétase.

**12.** Cristal d'enzyme réticulé avec un agent réticulant multifonctionnel, ledit cristal d'enzyme réticulé ayant une résistance à la protéolyse à la Pronase™ exogène, où ladite enzyme est l'estérase.

**13.** Cristal d'enzyme réticulé selon l'une quelconque des revendications 1 à 12, où ledit cristal est un microcristal ayant une section transversale d'environ $10^{-1}$ mm.

**14.** Cristal d'enzyme réticulé selon l'une quelconque des revendications 2, 4 ou 12, ledit cristal d'enzyme réticulé conservant au moins 91% de son activité initiale après incubation pendant trois heures en présence d'une concentration de Pronase™ qui force la forme non réticulée soluble de l'enzyme qui est cristallisée à former ledit cristal d'enzyme qui est réticulé pour conserver au plus 6% de son activité initiale dans les mêmes conditions où ladite enzyme est sélectionnée dans le groupe consistant en estérase, élastase et thermolysine.

**15.** Cristal d'enzyme réticulé selon l'une quelconque des revendications 1 à 4, où l'agent réticulant est glutaraldéhyde.

**16.** Dispositif comprenant un cristal d'enzyme réticulé selon l'une quelconque des revendications 1 à 5, et un moyen de retenue pour ledit cristal d'enzyme réticulé, ledit moyen de retenue consistant en un matériau qui permet le contact entre ledit cristal d'enzyme réticulé et un substrat sur lequel agit l'enzyme, le substrat étant présent dans un fluide.

**17.** Biocapteur pour détecter un analyte d'intérêt dans un fluide, comprenant :

a) un cristal d'enzyme réticulé selon l'une quelconque des revendications 1 à 15, où l'enzyme présente agit sur l'analyte d'intérêt ou sur un réactant dans une réaction dans laquelle participe l'analyte d'intérêt ;

b) un moyen de retenue pour ledit cristal d'enzyme réticulé, le moyen de retenue consistant en un matériau qui permet le contact entre ledit cristal d'enzyme réticulé et un fluide, ledit fluide contenant soit (1) l'analyte sur lequel agit l'enzyme ou (2) un réactant dans une réaction dans laquelle participe l'analyse.

**18.** Biocapteur selon la revendication 17, ledit biocapteur comprenant de plus un moyen de détection.

**19.** Utilisation d'un biocapteur selon la revendication 17 ou 18 pour la détection d'un analyte d'intérêt dans un fluide, où ledit analyte d'intérêt est sélectionné dans le groupe consistant en glucose, créatinine, urée, lactate, glucose-6-phosphate, saccharose, ATP, éthanol, acide acétique, acide formique, cholestérol, acide urique, méthotrexate, bioxyde de carbone, acides aminés, phosphates, pénicillines, nitrates, nitrites, sulfates et succinate.

**20.** Biocapteur selon la revendication 17, ledit cristal d'enzyme réticulé étant un cristal de luciférase, où la luciférase agit sur l'analyte d'intérêt ou sur un produit d'une réaction dans laquelle l'analyte d'intérêt participe et ledit moyen de retenue consiste en un matériau qui permet le contact entre ledit cristal de luciférase réticulé et un fluide, ledit fluide contenant soit (1) l'analyte sur lequel agit la luciférase ou (2) le produit d'une réaction dans laquelle participe l'analyte.

**21.** Dispositif extracorporel à utiliser pour modifier un composant d'un fluide comprenant :

a) un cristal d'enzyme réticulé selon l'une quelconque des revendications 1 à 15, où l'enzyme présente agit sur le composant ou sur un réactant dans une réaction dans laquelle participe le composant ; et

b) un moyen de retenue pour ledit cristal d'enzyme réticulé, ledit moyen de retenue consistant en un matériau qui permet un contact entre ledit cristal d'enzyme réticulé et un fluide, ledit fluide contenant soit (1) le composant sur lequel agit l'enzyme ou (2) le produit d'une réaction dans laquelle participe le composant.

**22.** Utilisation d'un dispositif extracorporel selon la revendication 1 pour modifier un composant d'un fluide où ledit composant est sélectionné dans le groupe consistant en asparagine, héparine, bilirubine, méthotrexate, acide aminé, urée et ammoniaque.

**23.** Dispositif à utiliser dans la production d'un produit sélectionné, comprenant une enzyme sous la forme d'un cristal d'enzyme réticulé selon l'une quelconque des revendications 1 à 15 et un moyen de retenue pour ledit cristal d'enzyme réticulé.

**24.** Méthode de production d'aspartame comprenant les étapes de :

a) combiner deux peptides et un cristal de thermolysine réticulé selon la revendication 2, le premier peptide ayant la formule Z-L-Asp et le seconde peptide ayant la formule L-Phe-OMe, dans des conditions appropriées pour la condensation des deux peptides par l'action dudit cristal de thermolysine réticulé, afin de produire ainsi un dipeptide partiellement protégé de la formule Z-L-Asp-L-Phe-Ome, où Z représente un groupe benzyloxycarbonyle, et

b) l'élimination dudit groupe benzyloxycarbonyle dudit dipeptide pour ainsi produire l'aspartame.

**Revendications pour l'Etat contractant suivant : ES**

**1.** Méthode pour la préparation d'un cristal d'enzyme réticulé avec un agent réticulant multifonctionnel, ledit cristal d'enzyme réticulé ayant une résistance à la protéolyse à la Pronase™ exogène où ladite enzyme est sélectionnée dans le groupe consistant en enzymes de pyridoxal phosphate, métalloenzymes, enzymes nécessitant CoA et sulfurases (PAPS), comprenant la cristallisation de l'enzyme avec ensuite réticulation du réseau cristallin avec un réactif multifonctionnel.

**2.** Méthode pour la préparation d'un cristal d'enzyme réticulé avec un agent réticulant multifonctionnel, ledit cristal d'enzyme réticulé ayant une résistance à la protéolyse à la Pronase™ exogène où ladite enzyme est sélectionnée dans le groupe consistant en thermolysine, nitrilase, aminocyclase et hydantoïnase, comprenant la cristallisation de l'enzyme avec ensuite réticulation du réseau cristallin avec un réactif multifonctionnel.

**3.** Méthode selon la revendication 2, où ladite enzyme est la thermolysine.

**4.** Méthode pour la préparation d'un cristal d'enzyme réticulé avec un agent réticulant multifonctionnel, ledit cristal d'enzyme réticulé ayant une résistance à la protéolyse à la Pronase™ exogène, où ladite enzyme est sélectionnée dans le groupe consistant en élastase, aminopeptidase, asparaginase, glutaminase, arginase, uréase, phosphatase alcaline, lipase, de préférence phospholipase, β-lactamase, ACL hydrolase, L-ACT hydrolase, arylsulfatase, glycosidase, de préférence β-galactosidase et β-fructosidase, pénicilline acylase, amidase, de préférence pénicilline amidase, estérase d'acide aminé, 5'-phosphodiestérase, nucléase et créatine désiminase, comprenant la cristallisation de l'enzyme avec ensuite réticulation du réseau cristallin avec un réactif multifonctionnel.

**5.** Méthode selon la revendication 4, où ladite enzyme est l'élastase.

**6.** Méthode selon la revendication 4, où ladite enzyme est l'asparaginase.

**7.** Méthode selon la revendication 4, où ladite enzyme est la lipase.

**8.** Méthode pour la préparation d'un cristal d'enzyme réticulé avec un agent réticulant multifonctionnel, ledit cristal d'enzyme réticulé ayant une résistance à la protéolyse à la Pronase™ exogène, où ladite enzyme est sélectionnée dans le groupe consistant . en déshydrogénase d'acide aminé, formiate déshydrogénase, lactate déshydrogénase, glutamate déshydrogénase, hydroxystéroïde déshydrogénase, glucose-6-pyruvate déshydrogénase, succinate déshydrogénase, glucose déshydrogénase, catalase, superoxyde dismutase, peroxydase, luciférase, tyrosinase, flavoenzyme, nitrate/nitrite réductase, oxydoréductase (NAD/P), oxydase et lyase, comprenant la cristallisation de l'enzyme avec ensuite réticulation du réseau cristallin avec un réactif multifonctionnel.

**9.** Méthode selon la revendication 8, où ladite oxydase est alcool oxydase, glucose oxydase, bilirubine oxydase, lactate oxydase, cholestérol oxydase ou oxydase d'acide L-aminé, et/ou ladite lyase est aldolase, L-aspartate 4-décarboxylase, phénylalanine ammonium lyase, anhydrase carbonique, nitrile hydratase, L-aspartase, fumarase ou héparinase.

**10.** Méthode pour la préparation d'un cristal d'enzyme réticulé avec un agent réticulant multifonctionnel, ledit cristal d'enzyme réticulé ayant une résistance à la protéolyse à la Pronase™ exogène, où ladite enzyme est sélectionnée dans le groupe consistant en kinase (ATP), de préférence créatine kinase, transaminase, glycosyl transférase et méthyl transférase (SAM), comprenant la cristallisation de l'enzyme avec ensuite réticulation du réseau cristallin avec un réactif multifonctionnel.

**11.** Méthode pour la préparation d'un cristal d'enzyme réticulé avec un agent réticulant multifonctionnel, ledit cristal d'enzyme réticulé ayant une résistance à la protéolyse à la Pronase™ exogène, où ladite enzyme est sélectionnée dans le groupe consistant en L-tryptophane synthétase et strictodine synthétase, comprenant la cristallisation de l'enzyme avec ensuite réticulation du -réseau cristallin avec un réactif multifonctionnel.

**12.** Méthode pour la préparation d'un cristal d'enzyme réticulé avec un agent réticulant multifonctionnel, ledit cristal d'enzyme réticulé ayant une résistance à la protéolyse à la Pronase™ exogène, où ladite enzyme est l'estérase, comprenant la cristallisation de l'enzyme avec ensuite réticulation du réseau cristallin avec un réactif multifonctionnel.

**13.** Méthode selon l'une quelconque des revendications 1 à 12, où ledit cristal est un microcristal ayant une section transversale d'environ 10 mm.

**14.** Méthode selon l'une quelconque des revendications 2, 4 ou 12, ledit cristal d'enzyme réticulé conservant au moins 91% de son activité initiale après incubation pendant trois heures en présence d'une concentration de Pronase™ qui force la forme non réticulée soluble de l'enzyme qui est cristallisée à former ledit cristal d'enzyme qui est réticulé pour conserver au plus 6% de son activité initiale dans les mêmes conditions où ladite enzyme est sélectionnée dans le groupe consistant en estérase, élastase et thermolysine.

**15.** Méthode selon l'une quelconque des revendications 1 à 4, où l'agent réticulant est glutaraldéhyde.

**16.** Dispositif comprenant un cristal d'enzyme réticulé selon l'une quelconque des revendications 1 à 5, et un moyen de retenue pour ledit cristal d'enzyme réticulé, ledit moyen de retenue consistant en un matériau qui permet le contact entre ledit cristal d'enzyme réticulé et un substrat sur lequel agit l'enzyme, le substrat étant présent dans un fluide.

**17.** Biocapteur pour détecter un analyte d'intérêt dans un fluide, comprenant :

a) un cristal d'enzyme réticulé selon l'une quelconque des revendications 1 à 15, où l'enzyme présente agit sur l'analyte d'intérêt ou sur un réactant dans une réaction dans laquelle participe l'analyte d'intérêt ;

b) un moyen de retenue pour ledit cristal d'enzyme réticulé, le moyen de retenue consistant en un matériau qui permet le contact entre ledit cristal d'enzyme réticulé et un fluide, ledit fluide contenant soit (1) l'analyte sur lequel agit l'enzyme ou (2) un réactant dans une réaction dans laquelle participe l'analyse.

**18.** Biocapteur selon la revendication 17, ledit biocapteur comprenant de plus un moyen de détection.

**19.** Utilisation d'un biocapteur selon la revendication 17 ou 18 pour la détection d'un analyte d'intérêt dans un fluide, où ledit analyte d'intérêt est sélectionné dans le groupe consistant en glucose, créatinine, urée, lactate, glucose-6-phosphate, saccharose, ATP, éthanol, acide acétique, acide formique, cholestérol, acide urique, méthotrexate, bioxyde de carbone, acides aminés, phosphates, pénicillines, nitrates, nitrites, sulfates et succinate.

**20.** Biocapteur selon la revendication 17, ledit cristal d'enzyme réticulé étant un cristal de luciférase, où la luciférase agit sur l'analyte d'intérêt ou sur un produit d'une réaction dans laquelle l'analyte d'intérêt participe et ledit moyen de retenue consiste en un matériau qui permet le contact entre ledit cristal de luciférase réticulé et un fluide, ledit fluide contenant soit (1) l'analyte sur lequel agit la luciférase ou (2) le produit d'une réaction dans laquelle participe l'analyte.

**21.** Dispositif extracorporel à utiliser pour modifier un composant d'un fluide comprenant :

a) un cristal d'enzyme réticulé selon l'une quelconque des revendications 1 à 15, où l'enzyme présente agit sur le composant ou sur un réactant dans une réaction dans laquelle participe le composant ; et

b) un moyen de retenue pour ledit cristal d'enzyme réticulé, ledit moyen de retenue consistant en un matériau qui permet un contact entre ledit cristal d'enzyme réticulé et un fluide, ledit fluide contenant soit (1) le composant sur lequel agit l'enzyme ou (2) le produit d'une réaction dans laquelle participe le composant.

**22.** Utilisation d'un dispositif extracorporel selon la revendication 1 pour modifier un composant d'un fluide où ledit composant est sélectionné dans le groupe consistant en asparagine, héparine, bilirubine, méthotrexate, acide aminé, urée et ammoniaque.

**23.** Dispositif à utiliser dans la production d'un produit sélectionné, comprenant une enzyme sous la forme d'un cristal d'enzyme réticulé selon l'une quelconque des revendications 1 à 15 et un moyen de retenue pour ledit cristal d'enzyme réticulé.

**24.** Méthode de production d'aspartame comprenant les étapes de :

a) combiner deux peptides et un cristal de thermolysine réticulé selon la revendication 2, le premier peptide ayant la formule Z-L-Asp et le seconde peptide ayant la formule L-Phe-OMe, dans des conditions appropriées pour la condensation des deux peptides par l'action dudit cristal de thermolysine réticulé, afin de produire ainsi un dipeptide partiellement protégé de la formule Z-L-Asp-L-Phe-Ome, où Z représente un groupe benzyloxycarbonyle, et

b) l'élimination dudit groupe benzyloxycarbonyle dudit dipeptide pour ainsi produire l'aspartame.

0.33
0.31
0.29
0.27
0.25
0.23
0.21
0.19
0.17
Absorbance (345nm)
CLEC Thermolysin
Soluble Thermolysin
0
5
10
15
Time (min)

FIG. 1

% Maximum Activity
0
20
40
60
80
100
4.5
5.5
6.5
7.5
8.5
9.5
10.5
pH
CLEC Thermolysin
Soluble Thermolysin

FIG. 2

100
80
60
40
20
0
% Maximum Activity
0.0
1.0
2.0
3.0
4.0
5.0
Time (days)
CLEC Thermolysin
Soluble Thermolysin


FIG. 3

% Maximum Activity
100
80
60
40
20
0
0.0
1.0
2.0
3.0
4.0
Time (days)
CLEC Thermolysin
Soluble Thermolysin

FIG. 4

% Max. Activity
100
80
60
40
20
0
30
60
90
Time (min)
Soluble Thermolysin

FIG. 4A

1.6
1.4
1.2
1.0
0.8
0.6
0.4
0.2
0.0
Absorbance (410nm)
CLEC Elastase
Soluble Elastase
0
5
10
15
Time (min)

FIG. 5

% Maximum Activity
100
80
60
40
20
0
0
30
60
90
120
150
180
Time (min)
CLEC Elastase
Soluble Elastase

FIG. 6

0.40
0.30
0.20
0.10
0.00
Absorbance (400nm)
0.0
2.0
4.0
6.0
8.0
10.0
Time (min)
CLEC Esterase
Soluble Esterase

FIG. 7

100
80
60
40
20
0
% Maximum Activity
0
30
60
90
120
150
180
Time (min)
CLEC Esterase
Soluble Esterase

FIG. 8

0.55
0.50
0.45
0.40
0.35
0.30
0.25
0.20
0.15
0.10
0.05
0.00
Absorbance (400nm)
0
10
20
30
40
50
60
70
80
90
Time (min)
CLEC Lipase
Soluble Lipase


FIG. 9

60
50
40
30
20
10
0
Fluorescence
0
30
60
90
120
Time (min)
CLEC Lysozyme
Soluble Lysozyme

FIG. 10

1.00
0.90
0.80
0.70
0.60
0.50
0.40
0.30
0.20
0.10
0.00
Absorbance (340nm)
0
5
10
15
20
25
30
35
40
45
Time (min)
CLEC Asparaginase
Soluble Asparaginase


FIG. 11